(19) Europäisches Patentamt
European Patent Office
Office européen des brevets

(11) Publication number: **0 116 713**
**A1**

(12) # EUROPEAN PATENT APPLICATION

(21) Application number: 83112756.8

(22) Date of filing: 19.12.83

(51) Int. Cl.³: C 07 D 311/92
C 07 D 409/04

(30) Priority: 27.12.82 US 453436
17.10.83 US 542825

(43) Date of publication of application:
29.08.84 Bulletin 84/35

(84) Designated Contracting States:
AT BE CH DE FR GB IT LI NL SE

(71) Applicant: SCHERING CORPORATION
2000 Galloping Hill Road
Kenilworth, New Jersey 07033(US)

(72) Inventor: Kreutner, William
69 Annin Road
West Caldwell New Jersey 07006(US)

(72) Inventor: Green, Michael John
43 Meadow Run Lane
Skillman New Jersey 08558(US)

(72) Inventor: Saksena, Anil Kumar
53 Beverly Road
Upper Montclair New Jersey 07043(US)

(72) Inventor: Shue, Ho-Jane
8 Hamilton Place
Pine Brook New Jersey 07058(US)

(74) Representative: Antony, Fritz, Dr. et al,
P.O. Box 601 Winkelriedstrasse 35
CH-6002 Lucerne(CH)

(54) **Novel labdane derivatives, process for the preparation thereof, and pharmaceutical compositions containing labdane derivatives.**

(57) This invention relates to a pharmaceutical composition eliciting an antiallergic and/or bronchodilatory effect, which comprises a compound of general formula I

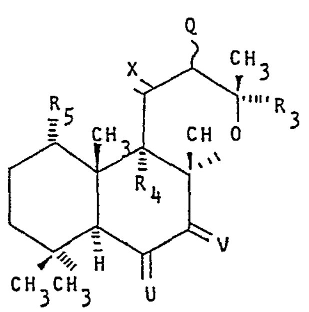

I

including the 5,6 and 6,7 dehydro derivatives, in the free form or in the form of its solvate,

wherein U is =O or $(H,R_1)$;

V is =O or $(H,R_2)$;

wherein $R_1$ is H or $OR_{11}$, wherein $R_{11}$ is H or acyl, $R_2$ is H, $OR_{11}$, $OSO_2R'$ or OR', wherein R' is alkyl; or when $R_1$ and $R_2$ are taken together they may form

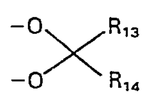

;

$R_3$ is hydrogen, alkyl, $CH_2OH$, CHO, $CO_2R_{15}$, $-CH=CR_{16}R_{17}$, $-C{\equiv}C-R_{18}$, $-CHOH-C{\equiv}C-R_{19}$;

$-CH=C=CHR_{19}$, $-CH=H-OR_{19}$, 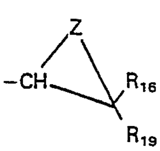

wherein Z is O or S, 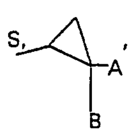 , $-CH=N-NDE$ or

$-CH(ZR_{20})_2$ wherein Z is defined as above;

$R_4$ is H or OH;

$R_5$ is OH; or when

$R_4$ and $R_5$ are taken together they may form

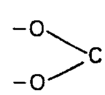 =Z wherein Z is defined as above; ./...

EP 0 116 713 A1

X is O or H'OR" wherein R" is H, carboxylic acyl or trifluoroacetyl;

Q is hydrogen, chlorine or bromine; or Q together with $R_3$ may form a bond between the carbon atoms of positions 12 and 13; whereby in the 5,6-dehydro-derivatives of formula I $R_1$ is H and in the 6,7-dehydro-derivatives of formula I $R_1$ and $R_2$ are H;

and wherein $R_{11}$, $R_{13}$, $R_{14}$, $T_{15}$, $R_{16}$, $R_{17}$, $R_{18}$, $R_{19}$, $R_{20}$, A, B, D, and E are as defined in the claims.

Part of the compounds of formula I are new compounds.

NOVEL LABDANE DERIVATIVES, PROCESS FOR THE PREPARATION THEREOF, AND PHARMACEUTICAL COMPOSITIONS CONTAINING LABDANE DERIVATIVES

U.S. Patent 4,118,508 describes a chemical compound, Coleforsin, which is said to possess hypotensive and positive inotropic effects.

U.S. Patent 4,134,986 describes polyoxygenated labdane derivatives which are also said to possess hypotensive and positive inotropic activities.

The compound Forskolin (7β-acetoxy-8,13-epoxy-1α,6β,9α-trihydroxylabd-14-en-11-one) is described in Arzneimittel-Forsch./Drug Res., 31, (II), 1248 (1981) as an adenylatecyclase activator.

J.C.S. Perkin I, 767 (1982) describes certain synthetic modifications of Forskolin.

This invention relates to novel labdane derivatives, process for the preparation thereof, and pharmaceutical

compositions containing labdane derivatives. These compounds and pharmaceutical compositions elicite an antiallergic and/or bronchodilatory effect and lower the intraocular pressure.

More particularly, this invention relates to pharmaceutical compositions eliciting an antiallergic and/or bronchodilatory effect, which comprise a compound of general formula I

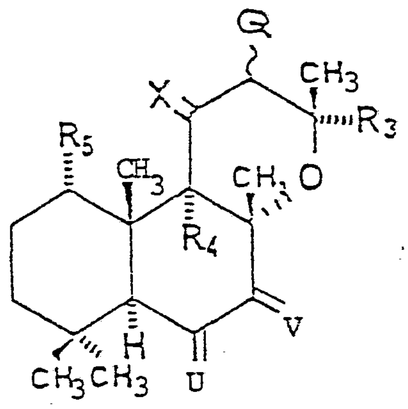

I

including the 5,6 and 6,7 dehydro derivatives, in the free form or in the form of its solvate,

wherein U is =O or $(H,R_1)$;

V is =O or $(H,R_2)$;

wherein $R_1$ is H or $OR_{11}$, wherein $R_{11}$ is H, carboxylic acyl having from 1 to 6 carbon atoms or

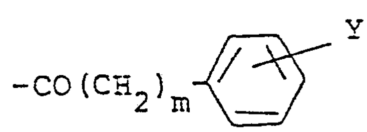

wherein m is 0, 1, 2 or 3 and Y is hydrogen, halogen, alkyl having from 1 to 6 carbon atoms, alkoxy having from 1 to 6 carbon atoms, alkylthio having from 1 to 6 carbon atoms, OH; $CF_3$, $NO_2$, CN, phenyl, benzyl,

phenoxy or $NR_aR_b$ wherein $R_a$ and $R_b$ are the same or different and are H or alkyl having from 1 to 6 carbon atoms;

$R_2$ is H, $OR_{11}$, $OSO_2R'$ or OR', wherein $R_{11}$ is defined as above and R' is alkyl having from 1 to 6 carbon atoms; or when

$R_1$ and $R_2$ are taken together they may form

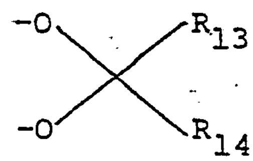

wherein $R_{13}$ and $R_{14}$ may be the same or different and are H, alkyl having from 1 to 6 carbon atoms, alkoxy having from 1 to 6 carbon atoms, alkenyl having from 2 to 6 carbon atoms, alkynyl having from 2 to 6 carbon atoms

or $-(CH_2)_m-$ 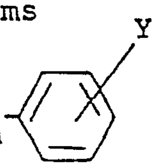 wherein m and Y are as defined above;

$R_3$ is hydrogen, alkyl having from 1 to 10 carbon atoms, $CH_2OH$, CHO, $CO_2R_{15}$ wherein $R_{15}$ is H or alkyl having from 1 to 6 carbon atoms,

$-CH=CR_{16}R_{17}$ wherein $R_{16}$ is H, halogen, alkyl having from 1 to 6 carbon atoms, CN, $\overset{O}{\overset{\|}{C}}(O)_nR_c$ (wherein n is θ or 1 and $R_c$ is H, alkyl having from 1 to 6 carbon atoms) phenyl or benzyl, $CHOHR_c$ or $C(OR_d)_2R_c$ (wherein $R_c$ is as defined above and $R_d$ is alkyl having

from 1 to 6 carbon atoms) $R_{17}$ is H, alkyl having from 1 to 6 carbon atoms, alkoxy having from 1 to 6 carbon atoms, benzyl, phenyl or halogen;

$-C{\equiv}C-R_{18}$ wherein $R_{18}$ is H, alkyl having from 1 to 12 carbon atoms, alkoxy having from 1 to 6 carbon atoms, $-CH_2-$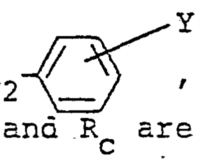, $CO_2R_c$ or 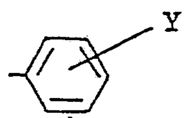 wherein Y and $R_c$ are defined as above;

$-CHOH-C{\equiv}C-R_{19}$ wherein $R_{19}$ is H, alkyl of from 1 to 6 carbon atoms, phenyl or benzyl;

$-CH{=}C{=}CHR_{19}$ or $-CH{=}N{-}OR_{19}$ wherein $R_{19}$ is defined as above

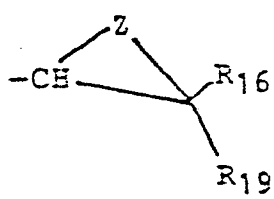 wherein Z is O or S, and

$R_{16}$ and $R_{19}$ are defined as above;

$-CH(ZR_{20})_2$ wherein Z is defined as above and $R_{20}$ is alkyl having from 1 to 6 carbon atoms, phenyl, benzyl or the two groups $R_{20}$ may together form $-(CH_2)_p-$ wherein p is 2 or 3;

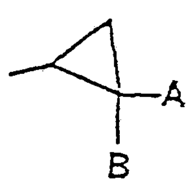 wherein A and B are H, halogen, alkyl having from 1 to 6 carbon atoms, phenyl, benzyl or $\overset{\overset{O}{\|}}{C}(O)_nR_c$ wherein n and $R_c$ are defined as above;

-CH=N-NDE wherein D and E are H, alkyl having from 1 to 6 carbon atoms, benzyl, phenyl, COG, $SO_2G$, or $CO_2G$ wherein G is alkyl having from 1 to 6 carbon atoms, benzyl or phenyl;

$R_4$ is H or OH;

$R_5$ is OH; or when

$R_4$ and $R_5$ are taken together they may form

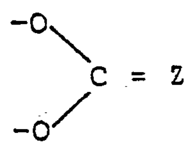

wherein Z is defined as above;

X is O or H/OR" wherein R" is H, carboxylic acyl having from 1 to 6 carbon atoms or trifluoroacetyl;

Q is hydrogen, chlorine or bromine; or Q together with $R_3$ may form a bond between the carbon atoms of positions 12 and 13;

whereby in the 5,6-dehydroderivatives of formula I $R_1$ is hydrogen and in the 6,7-dehydroderivatives of formula I $R_1$ and $R_2$ are hydrogen.

Part of the compounds defined are novel and will be discussed further below in more detail.

Of the compositions defined above those are of particular interest which contain a compound of formula I wherein

U is $(H,R_1)$ and V is $(H,R_2)$, wherein

$R_1$ and $R_2$ may be the same or different and are hydrogen or $OR_{11}$, wherein $R_{11}$ is defined as in claim 1 and Y is hydrogen;

$R_3$ is $-CH=CHR_{17}$

$-C\equiv CR_{18}$;

$-CHOH-C\equiv C-R_{19}$;

$-CH=C=CHR_{19}$;

$-CH=N-OR_{19}$;

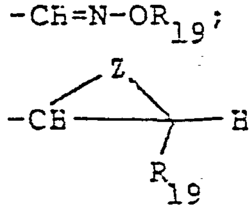 ; 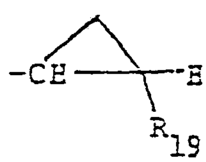 ;

wherein $R_{17}$, $R_{18}$ and $R_{19}$ are hydrogen, alkyl having from 1 to 6 carbon atoms, phenyl or benzyl;

$-CH=N-NHD$ wherein D is hydrogen, alkyl having from 1 to 6 carbon atoms, phenyl, benzyl, $SO_2G$ or $CO_2G$ wherein G is alkyl having from 1 to 6 carbon atoms;

$R_4$ is H or OH;

$R_5$ is OH;

X is O or (H,OH);

Q is hydrogen or chlorine;

the carbon atoms of positions 5,6 and 6,7 are connected by single bonds or

the carbon atoms of positions 5 and 6 are connected by a double bond, in which instance $R_1$ is |————————|. hydrogen and $R_2$ is preferably $OR_{11}$, wherein $R_{11}$ is defined as in claim 1 and Y is hydrogen, more particularly the compositions containing a compound of formula I wherein

U is $(H,R_1)$ and V is $(H,R_2)$ wherein

$R_1$ and $R_2$ may be the same or different and are hydrogen or $OR_{11}$, wherein $R_{11}$ is defined as above and Y is hydrogen:

$R_3$ is hydrogen, alkyl having from 1 to 6 carbon atoms, $-CH=CHR_{16}$ or $-C\equiv CR_{18}$ [wherein $R_{16}$ and $R_{18}$ are hydrogen, alkyl having from 1 to 6 carbon atoms, phenyl, benzyl or $CO_2R_c$ wherein $R_c$ is defined as in claim 1];

$R_4$ is H or OH;

$R_5$ is OH;

X is O or (H,OH);

Q is hydrogen or chlorine;

the carbon atoms of positions 5,6 and 6,7 are connected by single bonds or

the carbon atoms of positions 5 and 6 are connected by a double bond, in which instance $R_1$ is ├──┤ H and $R_2$ is preferably $OR_{11}$, wherein $R_{11}$ is defined as above and Y is hydrogen,

especially those wherein $R_4$ is hydrogen. Preferred areas are compositions containing a compound of formula I, wherein $R_1$ and $R_2$ are ├──┤ $OR_{11}$, wherein $R_{11}$ is H or acyl, preferably containing 1 to 3 carbon atoms, and/or $R_3$ is methyl, ethyl or vinyl.

Preferred compositions contain forskolin (=7β-acetoxy-8,13-epoxy-1α,6β,9α-trihydroxylabd-14-en-11-one),

7-desacetylforskolin (=8,13-epoxy-1α,6β,7β,9α-tetrahydroxylabd-14-en-11-one);

6-acetoxy-7-desacetylforskolin (=6β-acetoxy-8,13-epoxy-1α,7β,9α-trihydroxylabd-14-en-11-one),

8,13-epoxy-1α,6β,7β-trihydroxy-labd-14-en-11-one 7-acetate;

8,13-epoxy-1α,7β,9α-trihydroxy-labd-5(6)-ene-11-one-7-acetate;

8,13-epoxy-1α,6β,7β-trihydroxy-labd-14-en-11-one 6-acetate;

8,13-epoxy-1α,7β,9α-trihydroxy-labda-5(6),14-dien-11-one 7-acetate;

8,13-epoxy-1α,6β,7β-trihydroxy-labd-14-en-11-one;

8,13-epoxy-1α,6β,7β,11β-tetrahydroxy-labd-14-ene;

8,13-epoxy-1α,6β,7β,11β-tetrahydroxy-labd-14- ene-7-acetate;

12-chloro-8,13-epoxy-1α,7β-dihydroxy-labda-5(6), 14-dien-11-one 7-acetate;

15-nor-8,13-epoxy-1α,6β,7β,9α-tetrahydroxy-labdan-11-one;

15-nor-8,13-epoxy-1α,6β,7β,9α-tetrahydroxy-labdan-11-one-7-acetate;

8,13-epoxy-1α,7β,9α-trihydroxy-labda-5(6),14-diene 7-propionate, or

8,13-epoxy-1α,7β,9α-trihydroxy-labda-5(6),14-diene-11-one.

The compounds of formula I contained in the pharmaceutical compositions can be in their free form or in the form of their solvates, for example the hydrates.

When utilized herein the following terms will have the indicated meanings unless otherwise specified:

alkyl - straight and branched carbon chains having from 1 to 6 carbon atoms;

alkoxy and alkylthio - comprised of straight and branched carbon chains having from 1 to 6 carbon atoms which are singly bonded respectively to an oxygen or a sulfur atom;

halogen - fluorine, chlorine, bromine and iodine;

carboxylic acyl - the acyl portion derived from a straight or branched chain alkanoic acid having from 1 to 6 carbon atoms;

alkenyl - straight or branched carbon chains comprising one double bond and having from 2 to 6 carbon atoms:

alkynyl - straight and branched carbon chains comprising one to triple bond and having from 2 to 6 carbon atoms.

The following numbering system is utilized herein for formula I unless specified otherwise:

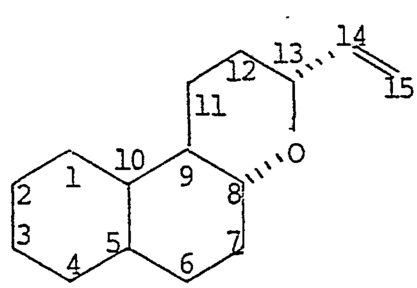

a dashed line (⁞⁞⁞) indicates that the substituent is projected below the plane of the paper and is denoted as α; a heavy line (◀) indicates that the substituent is projected above the plane of the paper and is denoted as β.

The compounds of formula I may exist as optical and/or geometiric isomers. The substituents at positions 6, 7, 11 and 12 (if not connected with the relevant carbon atoms by a double bond) can be in α- or β-position. Certain substituents, especially certain $R_3$-substituents may exist in isomeric forms (especially

$-CH=CR_{16}$, $R_{17}$, $-CHOHR_c$, $C(OR_d)_2R_c$, $-\overset{OH}{\overset{|}{C}}H-C\equiv CR_{19}$,

$-CH=C=CHR_{19}$, $-CH=N-OR_{19}$, 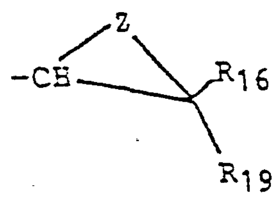 , 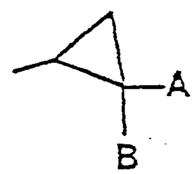

and -CH=N-NDE). In addition, when $R_3$ is hydrogen, the compound (I) exists in 2 epimeric forms. The invention contemplates all isomers, both in pure form and in admixture.

The compositions of this invention are considered to be no more toxic than compositions containing the active ingredients of formula I known in the art.

In the compositions of this invention the active compounds of formula I are combined with pharmaceutically acceptable carriers. The compositions are administered in well known pharmaceutical forms for oral and parenteral use.

The compounds can be administered in conventional oral dosage forms such as capsules, tablets, pills, powders, suspensions or solutions prepared with conventional pharmaceutically acceptable excipients and additives, using conventional techniques. Parenteral preparations, i.e. sterile solutions of suspensions are also made by conventional means. Inhalation administration can be in the form of a nasal or oral spray. Insufflation is also contemplated. Topical dosage forms can be creams, ointments, lotions and the like including ophthalmic preparations for the treatment of intraocular pressure. Other dosage forms which can be utilized are transdermal devices and suppositories.

The typical acceptable pharmaceutical carriers for use in the formulations described above are exemplified by: sugars such as lactose, sucrose, mannitol and sorbitol; starches such as corn starch, tapioca starch and potato starch; cellulose and derivatives such as sodium carboxymethyl cellulose, ethyl cellulose and methyl cellulose; calcium phosphates such as dicalcium phosphate and tricalcium phosphate; sodium sulfate; calucium sulfate, poly-vinylpyrrolidone, polyvinyl alcohol; stearic acid; alkaline earth metal stearates such as magnesium stearate and calcium stearate, stearic acid, vegetable oils such as peanut oil, cottonseed oil, sesame oil, olive oil and corn oil; non-ionic, cationic and anionic surfactants; ethylene glycol polymers; beta-cyclodextrin; fatty alcohols and hydrolyzed cereal solids; as well as other non-toxic compatible fillers, binders, disintergrants, buffers, preservatives, antioxidants,

lubricants, flavoring agents, and the like commonly used in pharmaceutical formulations.

The compositions of this invention can be used to treat allergy caused diseases, preferably for treating allergic chronic obstructive lung diseases. Chronic obstructive lung disease as used herein means disease conditions in which the passage of air through the lungs is obstructed or diminished such as is the case in asthma, bronchitis and the like.

From tests which measure a compound's inhibition of anaphylatic bronchospasm in sensitized guinea pigs having antigen induced bronchoconstriction can be concluded that the compounds of formula I elicit an anti-allergy effect. For example, the compound 7β-acetoxy-8,13-epoxy-1α-6β,9α-trihydroxy-labd-14-en-11-one was found to inhibit anaphylactic bronchospams in such test procedure when given at a dose of 1-3 mg/kg intravenously. Said compound was also found to inhibit allergen-induced histamine release from guinea pig and human sensitized tissue. It is concluded that the compounds of formula I defined above are effective non-adrenergic, non-anticholinergic, antianaphylactic and bronchodilator agents. When administered orally they are active at doses of from about 0.2 to 20 mg/kg of body weight; when administered parenterally, e.g., intravenously, the compounds are active at dosages of from about 0.1 to 3 mg/kg body weight; when administered by inhalation (aerosol or nebulizer) the compounds are active at dosages of about 0.1 to 10 mg per puff, one to four puffs may be taken every 4 hours.

The amount, route and frequency of administration of the compounds will be regulated accordingly to the judgement of the attending clinician considering such factors as age, condition and size of the patient as well as severity of the disease being treated. A typical recommended dosage regimen is oral administration of from 10 to 3000 mg/day, preferably 100 to 1000 mg/day, in two to four divided doses to achieve relief of the symptoms.

The following test procedure was utilized to assess the usefulness of the compounds of the invention for inhibiting antigen induced anaphylactic bronchospasm.

Male Hartley guinea pigs were sensitized with 5 mg ovalbumin injected intraperitoneally (i.p.) and 5 mg injected subcutaneously (s.c.) in 1 ml saline on day 1 and 5 mg ovalbumin injected i.p. on day 4. The sensitized animals were used 3-4 weeks later.

To measure anaphylactic bronchospasm, sensitized guinea pigs were fasted overnight and following morning anesthetized with 0.9 ml/kg i.p. of dialurethane (0.1 gm/ml diallylbarbituric acid, 0.4 gm/ml ethylurea and 0.4 gm/ml urethane). The trachea and jugular vein were cannulated and the animals were ventilated by a Harvard rodent respirator at 50 strokes/min with a stroke volume of 5 ml. A side arm to the tracheal cannula was connected to a Harvard pressure transducer to obtain a continuous measure of intratracheal pressure which was recorded on a Harvard polygraph. The recorder was calibrated so that a pressure of 50 mm Hg produced a 25 mm pen deflection. An increase in intratracheal pressure was taken as a measure of bronchoconstriction.

Each guinea pig was injected intravenously (i.v.) with 1 mg/kg dl-propranolol (HCl salt), 5 mg/kg indomethacin and 2 mg/kg mepyramine (maleate salt) given together in a volume of 1 ml/kg. Fifteen minutes later, the animals were challenged with antigen (0.5 per cent ovalbumin) delivered as an aerosol generated from a DeVilbiss Model 65 ultrasonic nebulizer and delivered through the tracheal cannula for 30 seconds. Bronchoconstriction was measured as the peak increase in intratracheal pressure occurring within 15 minutes after antigen challenge..

Bronchoconstriction was studied in four guinea pigs at a time. The effect of test compound given i.v. on anaphylactic bronchospasm is expressed as per cent reversal of the peak increase in intratracheal pressure.

Results from the above procedure for representative compounds of the invention are given below.

| Forskolin (derivative) | Percentage reversal at dose (mg/kg) |
|---|---|
| 8,13-Epoxy-1α,6β,7β,9α-tetrahydroxy-labd-14-en-11-one 7 acetate | 70 (1)<br>65 (0.3)<br>43 (0.1) |
| 8,13-Epoxy-1α,6β,7β,9α-tetrahydroxy-labd-14-en-11-one | 69 (1) |
| 8,13-Epoxy-1α,6β,7β,9α-tetrahydroxy-labd-14-en-11-one 6-acetate | 70 (1) |
| 8,13-Epoxy-1α,6β,7β,9α-tetrahydroxy-labdan-11-one 7-acetate | 30 (1) |
| 8,13-Epoxy-1α,6β,7β,9α,11β-pentahydroxy-labd-14-en 7-acetate | 33 (1) |
| 8,13-Epoxy-1α,6β,7β,9α,11β-pentahydroxy-labd-14-ene | 25 (1) |

Part of the compounds of the general formula I are new compounds. New compounds of this invention are compounds having the general formula I as defined above, including their solvates, wherein

when $R_4$ is hydrogen, 8,13-epoxy-1α,6β,7β,11α-tetrahydroxylabd-14-ene is excluded and

when $R_4$ is hydroxy or $R_4$ and $R_5$ taken together form the group 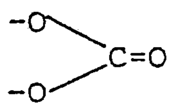, and the carbon atoms of positions (5,6) and (6,7) and (12,13) are connected by single bonds, compounds wherein $R_3$ is $CH=CH_2$, $CH_2CH_3$, CHO or 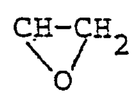.

are excluded.

The novel compounds of this invention can be specified in the following groups of compounds including their solvates:

-) compounds having the structural formula I as defined above wherein
$R_4$ is H;
8,13-epoxy-1α,6β,7β,11α-tetrahydroxylabd-14-ene being excluded;

-) compounds having the structural formula I as defined above provided that when $R_4$ is OH then $R_3$ is not $CH=CH_2$, $C_2H_5$, CHO or 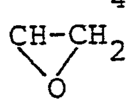.

-) compounds having the structural formula I as defined above wherein the carbon atoms of positions 5 and 6 are connected by a double bond;

$R_1$ is hydrogen; and

$R_5$ is hydroxy;

-) compounds having the structural formula I as defined above wherein the carbon atoms of positions 5 and 6 are connected by a double bond;

X is O or α-H/β-OH;

Q is H or Cl:

$R_1$ is H;

$R_2$ is $OR_{11}$;

$R_3$ is H, $CH_3$, $C_2H_5$ or $CH=CH_2$;

$R_4$ is H or OH; and

$R_5$ is OH.

-) compounds having the structural formula I as defined above wherein

$R_1$ and $R_2$ may be the same or different and are hydrogen or $OR_{11}$, wherein $R_{11}$ is defined as above and Y is hydrogen;

$R_3$ is hydrogen, alkyl having from 1 to 6 carbon atoms, $-CH=CHR_{16}$ or $-C{\equiv}CR_{18}$ [wherein $R_{16}$ and $R_{18}$ are hydrogen, alkyl having from 1 to 6 carbon atoms, phenyl, benzyl or $CO_2R_c$ wherein $R_c$ is defined as above;

$R_5$ is OH;

X is O or (H,OH);

Q is hydrogen or chlorine;

the carbon atoms of positions 5,6 and 6,7 are connected by single bonds or

the carbon atoms of positions 5 and 6 are connected by a double bond, in which instance $R_1$ is preferably H and $R_2$ is preferably $OR_{11}$, wherein $R_{11}$ is defined as in claim 1 and Y is hydrogen.

-) compounds having the structural formula I as defined above, wherein $R_1$ and $R_2$ may be the same or different and are H, or $OR_{11}$ and $R_{11}$ is H or carboxylic acyl having 1 or 2 carbon atoms, or
$R_1$ is =O in which case $R_2$ is $OR_{11}$;
$R_3$ is ethyl or vinyl;
$R_4$ is H;
$R_5$ is OH;
X is =O or H/OH; and
the carbon atoms of positions 5 and 6 are either connected by a single bond or by a double bond in which latter instance $R_1$ is H and $R_2$ is carboxylic acyloxy having 1 or 2 carbon atoms.

Among the novel compounds of this invention the following compounds are of particular interest:

8,13-epoxy-1α,6β,7β-trihydroxy-labd-14-en-11-one 7-acetate;

8,13-epoxy-1α,7β,9α-trihydroxy-labd-5(6)-en -11-one 7-acetate;

8,13-epoxy-1α,6β,7β-trihydroxy-labd-14-en-11-one 6-acetate;

8,13-epoxy-1α,7β,9α-trihydroxy-labda-5(6),14-dien 11-one 7-acetate;

8,13-epoxy-1α,6β,7β-trihydroxy-labd-14-en-11-one;

8,13-epoxy-1α,6β,7β,11β-tetrahydroxy-labd-14-ene;

8,13-epoxy-1α,6β,7β,11β-tetrahydroxy-labd-14-en-7-acetate;

12-chloro-8,13-epoxy-1α,7β-dihydroxy-labda-5(6),14-dien-11-one 7-acetate;

15-nor-8,13-epoxy-1α,6β,7β,9α-tetrahydroxy-labdan-11-one;

15-nor-8,13-epoxy-1α,6β,7β,9α-tetrahydroxy-labdan-11-one-7-acetate;

8,13-epoxy-1α,7β,9α-trihydroxy-labd-5(6),14-dien-7-propionate; or

8,13-epoxy-1α,7β,9α-trihydroxy-labda-5(6),14-dien-11-one.

The compounds, besides the activities discussed above, elicit, as can be concluded from test results, hypotensive, positive inotropic and adenylatecyclase activating effects.

The compounds of formula I can be prepared by standard procedures. A convenient starting material for preparing these compounds is 7β-acetoxy-8,13-epoxy-1α,6β,9α-trihydroxylabd-14-en-11-one, Forskolin, [J.C.S. Perkin I, 767 (1982)]. By appropriate combination of processes discussed below, the compounds can be prepared. Processes by which a mixture of isomers is obtained are followed by known steps to isolate the desired isomers. When carrying out some of the desired synthetic conversions it may be necessary to first protect other reactive sites which may be present in a molecule. Such protection is preferably accomplished by first forming a derivative at the site to be protected which derivative may be readily re-converted to the original functionality, if desired, after the particular synthetic conversion has been carried out. Examples of such protective conversions are esterification of alcohols and ketalization of ketones. Other such conversions will suggest themselves to those skilled in the art. Because such techniques are recognized as within the skill of the art, they are not included in the following description of the preparation of the compounds of formula I. Also solvates and salts of compounds of formula I are prepared according to known methods.

The processes, using forskolin or an appropriate derivative, thereof are as follows:

(1) Compounds wherein U is =O can be prepared by oxidizing a corresponding compound wherein U is

(H,OH). Compounds wherein V is =O can be prepared analogously. A suitable oxidizing agent is for example $MnO_2$ or pyridinium chlorochromate.

(2) Compounds wherein U is $(H,OR_{11})$, wherein $R_{11}$ is acyl (as defined above) can be prepared by acylation of a corresponding compound wherein $R_{11}$ is hydrogen. Compounds wherein V is $(H,OR_{11})$ wherein $R_{11}$ is acyl can be prepared analogously. The process can be carried out using for example the appropriate acylhalide (e.g. chloride) or acyl anhydride.

(3) Compounds wherein U and/or V is (H,OH) can be prepared by hydrolysis (e.g. using aqueous solution of NaOH) of the corresponding ester. By appropriate reaction conditions also the groups 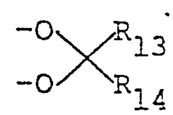 and 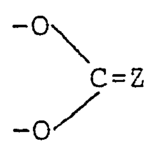 can be hydrolysed to form compounds containing hydroxy groups in positions 6 and 7 and/or 9 and 1.

(4) Compounds wherein U and/or V are (H,H) can be prepared by reduction of the corresponding compound wherein U and/or V are =O.

(5) Compounds wherein V is $(H,OSO_2R')$, wherein R' is alkyl having from 1 to 6 carbon atoms can be prepared by reacting a corresponding compound wherein V is (H,OH) with a reactive derivative of $R'SO_3H$ (e.g. $R'SO_2Cl$). This process is of interest for the preparation of compounds wherein V is $(H,OSO_2CH_3)$.

(6) Compounds wherein V is (H,OR') wherein R' is alkyl having from 1 to 6 carbon atoms, can be prepared by reacting a corresponding compound, wherein V is (H,OH) with the appropriate alkylhalogenide (R'hal), preferably the iodide. The reaction can for example be carried out in THF in the presence of sodium hydride. This process is of particular interest for the preparation of compounds wherein R' is methyl.

(7) Compounds wherein $R_1$ and $R_2$ together form

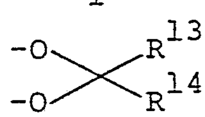

(wherein $R_{13}$ and $R_{14}$ are defined as above) may be prepared by reacting a corresponding compound wherein $R_1$=$R_2$=OH with an aldehyde or ketone. Such reactions are generally carried out using conditions whereby the reaction-produced water may be continuously removed.

(8) Compounds wherein the carbon atoms of positions 5,6 or 6,7 are connected by a double bond can be prepared by dehydration of the corresponding compound wherein said positions are connected by single bonds and U and/or V are (H,OH) or a reactive derivative thereof.

(9) Compounds wherein the carbon atoms of positions 12 and 13 are connected by a double bond can be prepared by removing $QR_3$ from a corresponding compound, wherein Q is H and $R_3$ is for example $-CH=NOR_{19}$ wherein $R_{19}$ is preferably hydrogen.

(10) Compounds wherein $R_4$ is hydrogen may be prepared by treatment of the corresponding 1,9-carbonate or 1,9-thiocarbonate (compound of formula I wherein $R_4$ and $R_5$ taken together form 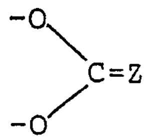 wherein Z is O or S) with zinc in acetic acid.

(11) Compounds wherein $R_4$ and $R_5$ taken together form 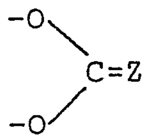, wherein Z is O or S can be prepared by converting the corresponding compound wherein $R_4$=$R_5$=OH by means of phosgene, carbonyl diimidazole or thiocarbonyl diimidazole, respectively.

(12) The compounds wherein X is (H,OH) may be prepared by reduction of a compound wherein X is oxo, e.g. by means of lithium aluminium hydride or catalytic hydrogenation. If, for example, a compound of formula I wherein $R_4$ is hydrogen, $R_5$ and $R_1$ are hydroxy and $R_2$ is hydroxy or acyloxy, is reacted with $LiAlH_4$ in $Et_2O$ at about 40°C the corresponding 1α,6β,7β,11-tetrahydroxy compound is obtained.

On the other hand, if for example a compound is catalytically hydrogenated (e.g. with hydrogen in the presence of $PtO_2$, wherein $R_1$ is hydroxy, $R_2$ is acyloxy, $R_4$ and $R_5$ together are forming a carbonate, the corresponding 11-hydroxy compound is obtained, wherein $R_1$, $R_2$, $R_4$ and $R_5$ are as in the starting material. If the starting compound has a double bond between position 12 and 13, this double bond is hydrogenated.

(13) Compounds wherein Q is chlorine or bromine can be prepared by chlorination or bromination of a corresponding compound wherein Q is hydrogen. For example a starting material wherein $R_4$ and $R_5$ are O(C=S)O can be reacted with $SOCl_2$ in pyridine to form the compound wherein Q is chlorine. If in the starting material U is (H,OH) the said reaction leads to a compound wherein Q is chlorine and the carbon atoms of positions 5 and 6 are connected by a double bond.

(14) Compounds wherein $R_3$ is hydrogen and X is (H,OH) can be prepared by hydrogenation of a corresponding compound wherein X is O and the carbon atoms of positions 12 and 13 are connected by a double bond.

(15) Compounds wherein $R_3$ is ethyl can be prepared by hydrogenation of the corresponding compound wherein $R_3$ is vinyl, e.g. by means of catalytic hydrogenation (e.g. using Pd/C or $PtO_2$).

(16) Compounds wherein $R_3$ is methyl can be prepared by desulfurizing a corresponding compound wherein $R_3$ is 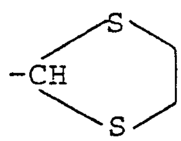, e.g. in a Ra/Ni suspension in ethanol.

(17) Compounds wherein $R_3$ is $CH_2OH$ can be prepared by reduction of a corresponding compound wherein $R_3$ is CHO.

(18) Compounds wherein $R_3$ is CHO can be prepared by ozonolysis of the corresponding compound wherein $R_3$ is vinyl. In the starting compound $R_4$ is preferably hydrogen or $R_4$ and $R_5$ together form the group 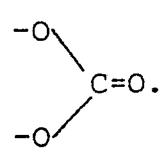.

(19) Compounds wherein $R_3$ is $CO_2R_{15}$ may be prepared by oxidizing a corresponding compound wherein $R_3$ is CHO followed by esterification if $R_{15}$ is alkyl having 1 to 6 carbon atoms.

(20) Compounds wherein $R_3$ is $-CH=CR_{16}R_{17}$ may be prepared by treating a corresponding compound wherein $R_3$ is CHO with a Wittig reagent, $\emptyset_3P=CR_{16}R_{17}$. Certain of the substituents, $R_{16}$ and $R_{17}$ may be modified or interconverted subsequent to the Wittig reaction, when desired.

(21) Compounds wherein $R_3$ is $C\equiv C-R_{18}$ may be prepared by treating a compound wherein $R_3$ is CHO with a reagent $\emptyset_3P^+CHBrR_{18}$ to produce a bromine containing intermediate which may be dehydrobrominated to produce the desired product.

(22) Compounds wherein $R_3$ is $C\equiv CH$ may be prepared by the addition of bromine to a corresponding compound wherein $R_3$ is $-CH=CH_2$ followed by didehydrobromination.

(23) Compounds wherein $R_3$ is $-CHOH-C\equiv C-R_{19}$ may be prepared from a compound wherein $R_3$ is CHO by treatment with a reagent, $MC\equiv C-R_{19}$, wherein M is a suitable metal such as lithium.

(24) Compounds wherein $R_3$ is $-CH=C=CHR_{19}$ may be prepared from a corresponding compound wherein $R_3$ is $-CHOH-C\equiv C-R_{19}$ by first esterifying the hydroxy group and treating the so obtained ester with aluminum chloride.

(25) Compounds wherein $R_3$ is $-CH=N-OR_{19}$ may be prepared from a compound wherein $R_3$ is CHO by treatment with a reagent of the formula $H_2N-OR_{19}$. The preparation is of particular interest for preparing compounds wherein $R_{19}$ is hydrogen or methyl. A suitable reaction medium is dry pyridine.

(26) Compounds wherein $R_3$ is 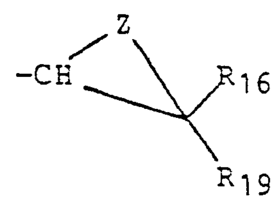

may be prepared from a compound wherein $R_3$ is $-CH=CR_{16}R_{19}$ by treatment with a peracid such as m-chloroperbenzoic acid to produce compounds wherein Z is oxygen, these compounds may be converted to corresponding compounds wherein Z is sulfur by treatment with potassium isothiocyanate.

(27) Compounds wherein $R_3$ is $-CH(ZR_{20})_2$ may be prepared from a compound wherein $R_3$ is CHO by treatment with an alcohol or thiol using standard procedures. The process is of particular interest for preparing compounds wherein

$R_3$ is 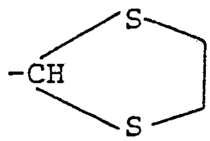 , whereby the corresponding compound ($R_3$:CHO) is reacted with ethanedithiol in dry $CH_2Cl_2$ with the addition of $BF_3$, $Et_2O$.

(28) Compounds wherein $R_3$ is 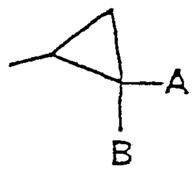 may be prepared by the addition of a dihalocarbene to a compound wherein $R_3$ is $-CH=CH_2$ or by the addition of a methylene carbene to a compound wherein $R_3$ is $-CH=CAB$. For the preparation of compounds wherein $R_3$ is cyclopropyl a compound wherein $R_3$ is $-CH=CH_2$ is preferably reacted with diazomethane.

(29) Compounds wherein $R_3$ is $-CH=N-NDE$ may be prepared by treating a compound wherein $R_3$ is CHO with a compound having the formula $H_2N-NDE$ using standard procedures.

The following examples illustrate the preparation of representative compounds of the invention. It is understood that all compounds of formula I can be prepared applying the disclosed and exemplified processes in appropriate order. Necessary adaptions of the reaction conditions are well known to those skilled in the art.

EXAMPLE I

8,13-Epoxy-1α,6β,7β,9α-tetrahydroxylabdan-11-one 7-acetate

Forskolin (30 mg) in MeOH (9 ml) with 10% Pd/C was hydrogenated at room temperature and atmospheric pressure for 2 hours. The catalyst was filtered off, and the solvent evaporated. Recrystallization from ether/hexane gave product, (16 mg) m.p. 242-244°C.

EXAMPLE II

8,13-Epoxy-1α,6β,7β,9α-tetrahydroxylabd-14-en-11-one 7-acetate 1,9-thiocarbonate

Forskolin (100 mg) was stirred with carbonyl diimidazole (130 mg) in $CH_2Cl_2$ (6 ml) at room temperature for 6 days. The solvent was evaporated and the residue chromotographed on a column of silica gel (10 g). Elution with 10% EtOAc/$CHCl_3$ gave product which was crystallized from ether/hexane (68 mg) m.p. 241-243°C.

EXAMPLE III

8,13-Epoxy-1α,6β,7β-trihydroxylabd-14-en-11-one 7-acetate

The thiocarbonate of forskolin (0.23 g) from Example II in AcOH (11.5 ml) was treated with Zn powder (0.66 g) and was heated to 120°C for 20 minutes. The zinc was filtered off hot, the filtrate poured into $H_2O$ (100 ml) and neutralized with NaOH. The product was extracted into ethyl acetate and chromotographed on a column of silica gel eluting with 10% EtOAc/$CHCl_3$ to give product which crystallized from acetone-hexane; (93 mg) m.p. 157.5-160°C.

EXAMPLE IV

8,13-Epoxy-1α,7ß,9α-trihydroxylabd-5(6)-en-11-one 7-acetate

Forskolin (1 g), benzyl bromide (0.82 ml), potassium iodide (1 g) and potassium carbonate (5 g) in 30 ml dry acetone was heated to 60°C for 18 hours. After cooling the precipitate was filtered off, the filtrate was evaporated and the residue was separated on a column of silica gel (100 g) eluting with 5% EtOAc/$CHCl_3$ to give 1α-benzylforskolin (0.15 g). 80 mg of this material was treated with thionyl chloride (0.08 ml) in pyridine (0.8 ml) for 4 hours at 0°C. The reaction was poured into water and the product was isolated by extraction with ethyl acetate. This product was hydrogenated ($PtO_2$ 0.5 g, EtOAc 15 ml) for 24 hours at room temperature after which it was purified on a column of silica gel eluting with 20% EtOAc/hexane to give product crystallized from acetane hexane (19 mg).

EXAMPLE V

8,13-Epoxy-1α,6ß,7ß-trihydroxylabd-14-en-11-one

9-Deoxyforskolin (0.44 g) prepared in Example III in methyl alcohol (21 ml) was treated with 5.28 ml of a 1.66% NaOH aqueous solution for 17 hours at room temperature. The resulting solution was neutralized with 2N HCl, the methanol was removed under reduced pressure and the product was taken into ethyl acetate. The organic layer was washed with water, dried ($MgSO_4$) and the solvent was evaporated to a residue which was purified on a column of silica gel (eluting with 40% EtOAc/hexane) to give product 0.15 g.

EXAMPLE VI

A. 8,13-Epoxy-1α,6ß,7ß-trihydroxylabd-14-en-11-one 7-benzoate

The product from Example V (50 mg) was treated in pyridine (0.5 ml) at 0°C with benzoyl chloride (0.018 ml) for 50 minutes. 2NHCl was added and then water; the product was isolated and purified on a column of silica gel eluting with 25% EtOAc/hexane. Crystallization from acetone-hexane gave product 33 mg m.p. 164.5°C.

B. 8,13-Epoxy-1α,6ß,7ß-trihydroxylabd-14-en-11-one 7-pentanoate

In a similar manner to Example VI A but substituting valeroyl chloride for benzoyl chloride, the product was obtained, m.p. 98-100°C.

C. 8,13-Epoxy-1α,6ß,7ß-trihydroxylabd-14-en-11-one 7-(2-methylpropanoate)

In a similar manner to Example VI A but substituting 2-methylpropanoyl chloride for benzoyl chloride, the product was obtained, m.p. 145.5-147.5°C.

EXAMPLE VII

Forskolin 1,9-carbonate

A stirred and cooled (ice bath) solution of Forskolin (1 g) in dry methylene chloride (10 ml) and pyridine (3 ml) was treated with a solution of phosgene in methylene chloride (3 ml; approx. 13% phosgene by weight). After 45 minutes the starting material was all

consumed. Methylene chloride and pyridine were removed *in vacuo* and the residue was distributed between methylene chloride (25 ml) and water (10 ml). The organic phase was separated and the aqueous phase extracted twice with methylene chloride. The combined organic extracts were dried ($Na_2SO_4$) and evaporated to dryness to provide a crystalline solid, 1.29 gms. A 100 mg. portion of this product was recrystallized from ethyl acetate/n-hexane, m.p. 121-123°C.

## EXAMPLE VIII

### 14,15-Dinor-8,13-epoxy-13-formyl-1α,6β,7β,9α-tetrahydroxylabdan-11-one 1,9-carbonate

A solution of Forskolin 1,9-carbonate (0.2 g) prepared as in Example VII in methylene chloride (4 ml) and methanol (3 ml) was cooled in acetone/dry ice bath. Ozone/oxygen was passed through this solution until excess ozone (indicated by blue coloration) was present (approx. 5-7 minutes). Excess ozone was driven off by passing argon through the solution and 0.5 ml dimethylsulfide was added. The reaction mixture was gradually allowed to warm to room temperature and after stirring 2 hours, evaporated to dryness *in vacuo*. The residue was dissolved in methylene chloride (15 ml) and washed with water. The methylene chloride solution was dried ($Na_2SO_4$) and evaporated to dryness to yield a crystalline product nearly homogenous by TLC. Yield: 0.190 g. A 20 mg. portion was recrystallized from ethyl acetate/n-hexane, m.p. 237-238°C.

EXAMPLE IX

15-Nor-8,13-epoxy-1α,6β,7β,9α-tetrahydroxy-14-hydroxyiminolabdan-11-one 7-acetate 1,9-carbonate

A solution of the aldehyde (0.4 g) prepared as in Example VIII in dry pyridine (5 ml) was cooled (ice bath) and treated with solid $NH_2OH.HCl$ (0.07 g) with stirring. After approximately 2 hours the reaction mixture was diluted with methylene chloride (25 ml) and treated with dilute $H_2SO_4$ (0.1N) until the aqueous phase was acidic. The methylene chloride layer was separated, and the aqueous phase was extracted once with methylene chloride. The combined methylene chloride extracts were washed once with water, dried ($Na_2SO_4$) and evaporated to dryness to provide a crystalline TLC homogenous product. Yield: 0.38 g. A small portion of this product (30 mg.) was recrystallized from chloroform to provide colorless cubes, m.p. 277-280°C.

EXAMPLE X

14,-E and Z-8,13-Epoxy-1α,6β,7β,9α-tetrahydroxylabd-14-en-11-one 7-acetate 1,9-carbonate 15-methylcarboxylate

A solution of the aldehyde (0.1 g) as prepared in Example VIII in methylene chloride (5 ml) was treated with the phosphorane ($Ph_3P=CH.COOMe$) (0.085 g; ca. 1.1 equiv.) and the reaction mixture refluxed for 2 hours. TLC showed a two-component mixture. Refluxing for an additional 2 hours did not change the product ratios by TLC. The methylene chloride was replaced by benzene (4 ml), additional phosphorane (0.04 g) was added and the reaction mixture again refluxed for 1 hour. The benzene was evaporated to dryness *in vacuo* and the residue subjected to chromatography on two 1 mm thick silica gel plates using 40% acetone/n-hexane as eluent:

| | |
|---|---|
| LESS POLAR COMPONENT A (Z-isomer) | 0.08 g. crystals m.p. 120-121°C |
| MORE POLAR COMPONENT B (E-isomer) | 0.04 g. crystals m.p. 214-216°C |

## EXAMPLE XI

### 8,13-Epoxy-1α,7ß,9α-trihydroxylabda-5(6), 14-diene-11-one 7-acetate 1,9-carbonate

To a stirred solution of forskolin 1,9-carbonate (0.87g,) from Example VII in dry $CH_2Cl_2$ (17.4 ml) was added diethylaminosulfurtrifluoride (0.5 ml) under a nitrogen atmosphere at 0°C. After stirring for 15 minutes, another portion of diethylaminosulfurtrifluoride (0.5 ml) was added and stirred for a further 30 minutes. EtOAc (200 ml) was added to the mixture and then washed immediately with saturated $NaHCO_3$ solution and water. The organic fraction was dried ($MgSO_4$) and evaporated in vacuo to dryness to yield a white solid, 0.79 g (95%). A portion of product was crystallized from EtOAc/hexane to give product m.p. 164-166°C.

## EXAMPLE XII

### a) 8,13-Epoxy-1α,7ß,9α-trihydroxy-labda-5(6), 14-diene-11-one and b) 8,13-Epoxy-1α,7ß,9α-trihydroxy-labd-5(6),14-diene-11-one 7 acetate

A mixture of 8,13-epoxy-1α,7ß,9α-trihydroxy-labd-5(6),14-diene-11-one 7-acetate 1,9-carbonate (0.78 g,), prepared as in Example XI, MeOH (40 ml) and NaOH solution (1.66% in $H_2O$, 5 ml) was stirred at room temperature for 3.5 hours. An additional NaOH solution (1.66% NaOH in $H_2O$, 5 ml) was added and stirred for a further 30 minutes. The mixture was neutralized with a few drops of HCl (2N) and the solution

was evaporated *in vacuo*. The residue was redissolved in EtOAc and washed with brine, dried ($MgSO_4$) and concentrated under reduced pressure to give a white solid, 0.64 g. Column chromatography (100 g silica gel, 40% EtOAc/$CHCl_3$) afforded 8,13-epoxy-1α,7ß,9α-trihydroxy-labda-5(6),14-diene-11-one 0.51 g (78%) which was crystallized from acetone/hexane, m.p. 179-181°C and 8,13-epoxy-1α,7ß,9α-trihydroxy-labd-5(6),14-diene-11-one 7-acetate 0.13 g (18%) which was crystallized from ethylacetate/hexane, m.p. 119-121°C.

EXAMPLE XIII

8,13-Epoxy-1α,7ß,9α-trihydroxy labda-5(6), 14-diene-11-one 7-propionate

To a cooled solution of 8,13-epoxy-1α,7ß,9α-trihydroxy-labd-5(6),14-diene-11-one (40 mg,) prepared as in Example XII in distilled pyridine (0.4 ml) was added propionic anhydride (0.2 ml) at 0°C (ice-water bath). The mixture was stirred at 0°C for 2.5 hours then at room temperature for 2 hours. The mixture was diluted with EtOAc (200 ml) and washed with 1N HCl, $H_2O$ and dried ($MgSO_4$). The solvent was evaporated *in vacuo* to a residue which was purified on thin layer chromatography plate (silica plates, 1mm, 40% EtOAc/hexane) to give product 26 mg (0.64 mmol, 56%). Crystallization from petroleum ether gave product 20 mg. m.p. 109.5°C-111°C.

EXAMPLE XIV

8,13-Epoxy-1α,7ß,9α-trihydroxy-labda-5(6), 14-diene-11-one 7-methylether

To a mixture of sodium hydride (60%, 5.7 mg) in THF (freshly distilled, 0.3 ml) was added $CH_3I$ (0.009 ml, 0.14 mmol) and 8,13-epoxy-1α,7β,9α-trihydroxy-labda-5(6),14-dien-11-one 1α-*p*-methoxybenzylether (prepared from the product of example XIIa by the method of example XXXI)

(25 mg, 0.07 mmol) in 0.3 ml of THF under a nitrogen atmosphere. The mixture was heated at 60°C for 30 minutes and quenched with a few drops of 1N HCl. Solvent was evaporated and the residue was separated on thin layer chromatography plates (silica gel, 40% EtOAC/Hexane). The 1α-p-methoxybenzyl group is removed by the method of example XXXI, to give the product.

EXAMPLE XV

8,13-Epoxy-1α,6ß,7α-trihydroxy-labda-14-en-11-one 7-methanesulfonate

A mixture of the product from Example V (50 mg) in pyridine (0.5 ml) and $CH_3SO_2Cl$ (25 μl) was stirred at room temperature for 1 hour. EtOAc (200 ml) was added to the mixture and washed immediately with 1N HCl and then with $H_2O$. The organic phase was dried ($MgSO_4$) and evaporated *in vacuo* to give a residue 54 mg. Purification on silica gel thin layer chromatography plates, (10% EtOAc/$CHCl_3$) yielded product 33 mg (0.073 mmol, 54%) m.p. 90-92°C.

EXAMPLE XVI

8,13-Epoxy-1α,7ß,9α,11ß-tetrahydroxy labda-5(6),14-diene

A mixture of the product from Example VII (forskolin 1,9-carbonate) (90 mg, 0.215 mmol) in THF (1 ml) ethylether (3.6 ml) and lithium aluminum hydride (45 mg, 1.18 mmol) was heated at 40°C under a nitrogen atmosphere for 1 hour.

To the cooled mixture (in ice-water bath) was added 50 ml $Et_2O$ and 0.2 ml of saturated $Na_2SO_4$ solution and stirred for 0.5 hour. The white precipitate was filtered off and washed with $Et_2O$ (50 ml). The filtrate was evaporated under reduced pressure to give a white residue 80 mg. This residue was purified on silica gel thin layer chromatography plates, (40% EtOAc/Hexane) to yield product 28 mg (0.079 mmol, 37%). Crystallization from EtOAc/Hexane gave product 22 mg. m.p. 120-126°C.

## EXAMPLE XVII

### 8,13-Epoxy-1α,6ß,7ß,11ß-tetrahydroxy-labd-14-ene

A mixture of product from Example III (9-deoxyforskolin) (15 mg, 0.14 mmol) in $Et_2O$ (3.0 ml) and lithium aluminum hydride (25 mg, 0.66 mmol) was heated to 40°C under a nitrogen atmosphere for 1.5 hours. The mixture was diluted with $Et_2O$ (50 ml) and cooled in ice-water bath. To the cooled mixture was dropwise added saturated $Na_2SO_4/H_2O$ solution (1 ml) and stirred for 30 minutes. The white precipitate was filtered off and washed with $Et_2O$ (50 ml). The organic solution was concentrated *in vacuo* to give a residue 44 mg. Purification of the residue on alumina TLC plate (250μ, 5% EtOAc-1% MeOH -94% $CHCl_3$) yielded product 23 mg (0.5 mmol, 46%) which crystallized from EtOAc/hexane, 21 mg m.p. 189-190°C.

## EXAMPLE XVIII

### 8,13-Epoxy-1α,6ß,7ß,11ß-tetrahydroxy-labd-14-ene 7-acetate

To a solution of crude product (94 mg, 0.265 mmol), prepared as in Example XVII, in pyridine (0.94 ml)

and $CH_2Cl_2$ (0.14 ml) was added acetic anhydride (0.135 ml, 1.32 mmol). The mixture was stirred at room temperature overnight, $H_2O$ (100 ml) was added to quench the reaction and product was extracted with EtOAc. The organic layer was washed with 1N HCl, $H_2O$ and dried ($MgSO_4$). The solvent was evaporated in vacuo to give a residue. Column chromatography on silica gel, (2% MeOH/$CHCl_3$) gave product 49 mg (47%) m.p. 132-136°C.

## EXAMPLE XIX

### 15-Nor-8,13-epoxy-1α,6ß,7ß,9α-tetra-hydroxy-14-dithioethylene-labdan-11-one-7-acetate; 1,9-carbonate

A solution of the product from Example VIII (100mg) in 5 ml dry $CH_2Cl_2$ containing 40 µl ethanedithiol was cooled to -78°C under $N_2$. To this was added 60 1 of $BF_3.Et_2O$ and the reaction was allowed to stir at this temperature for 1 hour. The reaction mixture was quenched with water and extracted with $CH_2Cl_2$. The organic extract was washed with dilute $NH_4OH$ solution dried ($Na_2SO_4$) and the solvent removed, to give the product (purified by preparative TLC eluting with 50% EtOAc/hexane) 80 mg, m.p. 285-287° C (d).

## EXAMPLE XX

### 15-Nor-8,13-epoxy-1α,6ß,7ß,9α-tetra-hydroxy-14-dithioethylene-labdan-11-one

A solution of the product from Example XIX (32.1 mg) in methanol (6.0 ml) was treated with 10% NaOH (1.5 ml). The reaction was allowed to stir for 16 hours. Oxalic acid (112.5 mg) was added and the product was extracted with $CH_2Cl_2$, separated and dried. Solvent removed to give the product 30 mg.

EXAMPLE XXI

15-Nor-8,13-epoxy-1α,6ß,7ß,9α-tetrahydroxy-14-dithioethylene-labdan-11-one-7-acetate

The product from the Example XX (69.5 mg) was acylated in dry pyridine (0.7 ml), with $Ac_2O$ (73.4 1) and dry $CH_2Cl_2$ (0.7 ml) in a similar manner as in Example XXIII. Purification by preparative TLC, eluting with EtOAc/ $MeOH/CHCl_3$ (5:1:94), afforded product 38.4 mg white solids m.p. 216-218°C (d).

EXAMPLE XXII

15-Nor-8,13-epoxy-1α,6ß,7ß,9α-tetrahydroxy-labdan-11-one

The product from Example XXI (165 mg) was desulfurized with 9 ml Ra/Ni suspension in 16 ml ethanol. The mixture was allowed to stir at room temperature for 16 hours. After filtration and washing with ethanol, the solvent was removed to give 112 mg. of the product. Recrystallization from methanol afforded white crystals of the product m.p. 191-192°C.

EXAMPLE XXIII

15-Nor-8,13-epoxy-1α,6ß,7ß,9α-tetrahydroxy-labdan-11-one-7-acetate

The product from Example XXII (91 mg) was dissolved in 0.8 ml dry pyridine and was treated with a solution of acetic anhydride (0.093 ml) in dry methylene chloride (0.8 ml). The reaction mixture was allowed to stir at room temperature under $N_2$ for 16 hours. The mixture was distributed between water and methylene chloride, the organic layer was washed with dilute HCl, dried and the solvent removed to give the product 87.4 mg. This crude product, purified by preparative TLC, eluting with 2% $MeOH/CHCl_3$, gave 40 mg crystalline product m.p. 274-276°C.

EXAMPLE XXIV

14,15-Dinor-13-cyclopropyl-8,13-epoxy-1α,6ß,7ß,9α-tetrahydroxy-labadan-11-one-7-acetate

A solution of forskolin (100 mg) in dry THF (1.0 ml) was treated with diazomethane in ether solution (3 ml) at 0°C. A small amount of $Pd(OAc)_2$ was added and the reaction mixture was stirred at this temperature for 15 minutes. The product was isolated and purified by preparative TLC, eluting with 5:1:94 ratio of EtOAc:MeOH:$CHCl_3$. Crystallization from $CHCl_3$ gave the product 65 mg, white crystals m.p. 253-256°C.

EXAMPLE XXV

14,15-Dinor-13-cyclopropyl-8,13-epoxy-1α,6ß,7ß-trihydroxy-labadan-11-one-7-acetate

9-Deoxyforskolin (100 mg) prepared as in Example III was cyclopropanated in a similar manner as in Example XXIV. The product was purified on a column of silica gel, eluting with 20% EtOAc/$CHCl_3$, and was further purified by preparative TLC, eluting with 30% EtOAc/$CHCl_3$, to give the product 53.2 mg. Crystallization from diisopropylether gave m.p. 205-205°C.

EXAMPLE XXVI

8,13-Epoxy-1α,6ß,7ß-trihydroxy-labd-14-en-11-one 6-acetate

A mixture of product from Example II (500 mg, 1.4 mmol), acetic acid (25 ml) and Zn powder (1.44 g) was heated at 120°C under a nitrogen atmosphere. After 30 minutes, another portion of Zn powder (1.44 g) was added and the mixture heated for a further 30 minutes. After cooling, the zinc was filtered off and the filtrate was concentrated *in vacuo* to a residue which was redissolved in EtOAc (300 ml), washed with water and dried ($MgSO_4$). The organic solvent was evaporated *in*

vacuo and the residue was chromatographed on a column of silica gel eluting with 10% EtoAc/$CHCl_3$ to give 9-desoxy forskolin 308 mg and product 40 mg. A 30 mg portion of the product was crystallized from acetone-hexane to give m.p. 190-192°C.

EXAMPLE XXVII

8,13-Epoxy-1α,7ß-dihydroxy-labda-5(6),14-diene-11-one 7-acetate

To a solution of the product from Example XI (70 mg 0.167 mmol) in acetic acid (3.5 ml) was added Zn powder (0.21 g). The mixture was heated to 120°C under a nitrogen atmosphere for 1.5 hours. EtOAc (100 ml) was added and the Zn was filtered off. The filtrate was poured into $H_2O$ (50 ml), neutralized with NaOH and the product was extracted into ethyl acetate. Chromatograph on a column of silica gel eluting with 30% EtOAc/hexane gave the product which was crystallized from ethyl acetate-hexane (14 mg).

EXAMPLE XXVIII

12-Chloro-8,13-epoxy-1α,7ß-dihydroxy-labda-5(6),14-diene-11-one 7-acetate

A mixture of the product from Example II (140 mg, 0.309 mmol) in pyridine (2.1 ml) and $SOCl_2$ (0.21 ml) was stirred at room temperature for 20 minutes. Water was added to the mixture and neutralized with 2NHCl. The product was extracted into ethyl acetate and, after work up, was chromatographed on silica TLC plates (1mm, 10% EtOAc/$CHCl_3$) to give product 20 mg, m.p. 103-105°C.

EXAMPLE XXIX

8,13-Epoxy-1α,7β,9α-trihydroxy-labda-5(6),14-diene-11-one 7-butyrate

A solution of product Example XIIa (44 mg, 0.114 mmol) in pyridine (0.4 ml) and butyric anhydride (0.2 ml) was stirred at 0°C for 24 hours. $H_2O$ was added at 0°C to quench the reaction and the mixture was diluted with EtOAc (200 ml). The organic solution was washed with 0.1N HCl, saturated $NaHCO_3$ solution, water and dried ($MgSO_4$). The solvent was evaporated *in vacuo* to give a residue (45 mg). Purification on silica TLC plates (1mm, 40% EtOAc-hexane) yielded product 26 mg (0.061 mmol, 54%) which was crystallized from petroleum ether to give m.p. 75-77°C.

EXAMPLE XXX

8,13-Epoxy-1α,6β,7β,9α-tetrahydroxy-labd-14-en-11-one 6,7-ethylorthoacetate

To a solution of 8,13-epoxy-1α,6β,7β,9α-tetrahydroxy-labd-14-en-11-one (800 mg, 0.217 mmol) in dry DMSO (7.05 ml) was added *p*-toluenesulfonic acid (63.5 mg) and triethylorthoacetate (1.53 ml). The mixture was stirred at room temperature for 2.3 hours, 5% aqueous $Na_2CO_3$ (100 ml) was added and the product was extracted with ethyl acetate. The combined EtOAc extracts were washed with water, dried ($MgSO_4$) and evaporated *in vacuo* to give product (930 mg, 0.272 mmol, 98%). A 100 mg portion of product was crystallized from EtOAc/hexane to give product m.p. 190.5-193°C.

## EXAMPLE XXXI

8,13-Epoxy-1α,9α-dihydroxy-labda-6(7),14-dien-11-one

Forskolin (1 g), p-methoxybenzyl chloride (1.0ml) potassium iodide (1 g) and potassium carbonate (5 g) in 30 ml dry acetone was heated at 60°C for 18 hours. After cooling, the precipitate was filtered off, the filtrate evaporated and the residue was separated on a column of silica gel (100 g) eluting with 10% EtOAc/$CHCl_3$ to give 1α-p-methoxybenzyl forskolin (0.4 g) and 0.8 g of a mixture of 1α-p-methoxybenzyl forskolin and 1α-p-methoxybenzyl-6-acetate-7-deacetyl forskolin. This mixture (0.8 g) was treated with MeOH (43.2 ml) and 1.66% NaOH-$H_2O$ (10.8 ml) at room temperature overnight. The MeOH was evaporated *in vacuo* and the residue was dissolved in EtOAc (300 ml), washed with water and dried ($MgSO_4$). Purification on a silica gel column yielded 1α-p-methoxy benzyl-7-deacetyl forskolin (0.63 g). This product was treated with toluene (20 ml), thiocarbonyldiimidazole (765 mg) and N,N-dimethylaminopyridine (63 mg) at 100°C under a nitrogen atomosphere for 24 hours. After evaporating off the solvent in vacuo, the residue was chromatographed on a column of silica gel (70 g, 20% EtOAc/hexane) to give 1α-p-methoxybenzyl-6ß,7ß-thiocarbonate 7-deacetyl forskolin (0.59 g). A portion of this product (250 mg) was heated in the presence of 1,3-dimethyl-2-phenyl-1,3-diazo-2-phosphacyclopentane (1.25 ml) at 50°C under an argon atmosphere in a reacti-vial for 1 day. Chromatography on a silica gel column (300 g, 15% EtOAc/hexane) gave 8,13-epoxy-1α,9α-dihydroxy-labda-

6(7),14-diene-11-one 1α-p-methoxy benzyl ether (62 mg). This product was treated with $CH_2Cl_2$: $H_2O$ (18:1) (3.0 ml) and DDQ (68.1 mg) at room temperature for 1 day. A further portion of DDQ (25 mg) was added and stirred at room temperature for a further day. The mixture was then filtered through celite, washed with acetone and the product purified on a silica gel columm (50 g, 30% EtOAc/hexane) to give 8,13-epoxy-1α,9α-dihydroxy-labda-6(7),14-diene-11-one 1α-p-methoxybenzoate (60 mg). This product (60 mg) was hydrolyzed with 1.66% NaOH-$H_2O$ (0.72 ml) in the presence of MeOH (3.0 ml) for 28 hours at room temperature. The resulting solution was neutralized with 2N HCl and the methanol was removed under reduced pressure. The product was dissolved in EtOAc, washed with water, dried ($MgSO_4$), evaporated in vacuo to a residue and purified on a column of silica gel (100 g, 10% EtOAc/hexane) to give product 40 mg. A 20 mg portion was crystallized from n-hexane, to give product m.p. 92-93°C.

## EXAMPLE XXXII

## 8,13-Epoxy-1α,9α-dihydroxy-labda-5(6),14-diene-7,11-dione

To a solution of the product from Example XIIa (50 mg) in $CHCl_3$ (3.0 ml) was added activated $MnO_2$ (50 mg, 0.575 mmol). The mixture was stirred at room temperature and additional activated $MnO_2$ (50 mg) was added on each of the following 2 days. On the third day, the mixture was diluted with $CHCl_3$ (100 ml) and filtered through a celite pad which was washed with additional $CHCl_3$ (50 ml). The filtrate was concentrated in vacuo to give a residue 45 mg. Chromatography on a silica gel column (40% EtOAc-$CHCl_3$) yielded product 14 mg (0.04 mmol, 28%). Crystallization from EtOAc/hexane gave product 10 mg, m.p. 205-208°C.

## EXAMPLE XXXIII

### 14,-E and Z-8,13-epoxy-1α,6ß,7ß-trihydroxy-labd-14-en-11-one 7-acetate 15-methylcarboxylate

A solution of 14,15-dinor-8,13-epoxy-13-formyl-1α,6ß,7ß-trihydroxy-labdan-11-one 7-acetate (obtained from ozonolysis of 9-deoxyforskolin, 100 mg as in Example XXXIV) in $CH_2Cl_2$ was treated with the phosphorane ($Ph_3P{=}CH.COOMe$; 90 mg). The reaction mixture was stirred at room temperature for 48 hours. The methylene chloride was evaporated in vacuo to provide a gummy residue which was chromatographed on silica gel TLC plates using 40% ethyl acetate/n-hexane as eluent to give the less polar Z-isomer as a colorless amphorphous solid (17.6 mg) and the more polar E-isomer as a colorless amphorphous solid (47.0 mg).

## EXAMPLE XXXIV

### 14,15-Dinor-8,13-epoxy-13-formyl-1α,6ß,7ß-trihydroxy-labdan-11-one 7-acetate

Ozone was passed through a cooled solution (bath temp. -70°) of 9-deoxy forskolin (50 gm) in methylene chloride (4 ml) and methanol (3 ml) until the blue coloration persisted. Excess ozone was flushed out with nitrogen followed by addition of 0.1 ml of $Me_2S$. The solution was gradually allowed to warm to room temperature (approx. 45 minutes) after which the solvents were evaporated in vacuo to provide a gummy residue. It was dissolved in $CH_2Cl_2$, the solution dried over $Na_2SO_4$ and evaporated to dryness. The so obtained aldehyde which was almost pure (TLC; solvent system 20% EtOAc/chloroform), was used as such in the following reactions.

EXAMPLE XXXV

15-Nor-8,13-epoxy-1α,6ß,7ß-trihydroxy-14-methoximino-labdan-11-one 7-acetate

A solution of 14,15-dinor-8,13-epoxy-13-formyl-1α,6ß,7ß-trihydroxy-labdan-11-one 7-acetate [as obtained from ozonolysis of 9-deoxy-forskolin, (50 mg) in Example XXXIV] in dry pyridine (2 ml) was treated with $NH_2OCH_3.HCl$ (12 mg). The mixture was stirred at room temperature for 2 hours after which, the pyridine was removed in vacuo. The resulting solid was distributed between $CH_2Cl_2$ and water. After separation of the organic phase, the aqueous phase was extracted once more with $CH_2Cl_2$. The combined methylene chloride extracts were dried over $Na_2SO_4$ and evaporated to dryness to provide a gummy solid which was subjected to chromatography on silica gel TLC plates using 20% ethyl acetate in chloroform as eluent.. Extraction of the major band with ethyl acetate provided the pure title compound as a colorless amorphous solid.

EXAMPLE XXXVI

14,15-Dinor-8,13-epoxy-1α,6ß,7ß,9α-tetrahydroxy-labd-12-en-11-one 7-acetate 1,9-carbonate

A solution of the product from Example IX (0.665 g) in $CH_2Cl_2$ (60 ml) was treated with carbonyl diimidazole (0.266 g) for 2 hours at room temperature. The organic solution was washed with $H_2O$, dried ($Na_2SO_4$) and evaporated to a solid residue. Purification by preparitive TLC (eluent 20% EtOAc/$CHCl_3$) gave the product (0.45 g.) m.p. 264-5°C after crystallization from EtOAc/hexane.

## EXAMPLE XXXVII

### 14,15-Dinor-8,13-epoxy-1α,6β,7β,9α-tetrahydroxy-labd-12-en-11-one

A solution of the product from Example XXXVI (0.1 g) in MeOH (8 ml) was treated with 10% NaOH (4 ml) for 15 hours at room temperature. Aqueous oxalic acid was added and the product was extracted into $CH_2Cl_2$. The organic solution was washed with water, dried ($Na_2SO_4$) and evaporated to give the product (0.094 g).

## EXAMPLE XXXVIII

### 14,15-Dinor-8,13-epoxy-1α,6β,7β,9α-tetrahydroxy-labd-12-en-11-one 7-acetate

The product from Example XXXVII (0.06 g) in dry pyridine (0.6 ml) and $CH_2Cl_2$ (0.8 ml) was treated with acetic anhydride (0.06 ml) for 20 hours at room temperature. Water was added and after 1 hour the reaction mixture was evaporated to a residue that was purified by preparitive TLC (eluent 20% EtOAc/hexane) to give the product (0.03 g), m.p. 224-5°C after crystallization from EtOAc/hexane.

## EXAMPLE XXXIX

### 8,13-Epoxy-1α,6β,7β,trihydroxy-labd-14-en-11-one 6,7-acetonide

The product from Example V (0.1 g) in dry acetone (10 ml) was treated with trimethylsilyl chloride (0.1 ml) at room temperature. After 15 hours a further portion of trimethylsilyl chloride (0.1 ml) was added and after another 15 hours the reaction was diluted with saturated $NaHCO_3$ solution. The product was extracted into $CH_2Cl_2$ and the organic extract was washed with $H_2O$, dried ($Na_2SO_4$) and evaporated to a residue that was purified

by preparitive TLC (eluent 10% EtOAc/$CHCl_3$) to give the product (0.045 g), m.p. 146-7°C from EtOAc/hexane.

EXAMPLE XL

13-Epi-14,15-dinor-8,13-epoxy-1α,6ß,7ß,9α,11-pentahydroxy-labdane 1,9-carbonate 7-acetate

The product from Example XXXVI (0.1 g) in EtOH (10 ml) was hydrogenated at 50 psi at room temperature for 36 hours in the presence of $PtO_2$ (0.1 g). The catalyst was then filtered off, the filtrate evaporated to a residue which was purified by preparitive TLC to give the product (0.067g).

EXAMPLE XLI

13-Epi-14,15-dinor-8,13-epoxy-1α,6ß,7ß,9α,11-pentahydroxy-labdane 7-acetate

A solution of the product from Example XL (0.067g) in MeOH (5 ml) was stirred overnight at room temperature with 10% NaOH solution (2 ml). Aqueous oxalic acid was added and the product extracted into $CH_2Cl_2$. The organic layer was washed with $H_2O$, dried ($Na_2SO_4$) and evaporated to a residue of 13-epi-14,15-dinor-8,13-epoxy-1α,6ß,7ß,9α,11-pentahydroxy-labdane. This material was dissolved in pyridine (0.7 ml) and $CH_2Cl_2$ (0.7 ml) and treated with $Ac_2O$ for 20 hours at room temperature. Water was added and the product extracted into $CH_2Cl_2$; the organic extract was washed with $H_2O$, dried ($Na_2SO_4$) and evaporated to a residue which was purified by preparitive TLC (eluent EtOAc/MeOH/$CHCl_3$-5/1/94) to give the product (0.024 g), m.p. 234-240°C from EtOAc/hexane.

EXAMPLE XLII

Capsules

| Ingredients | mg/Capsule | mg/Capsule |
|---|---|---|
| 8,13-epoxy-1α,7β,9α-trihydroxy-labda-5(6),14-dien-11-one 7 acetate | 50 | 100 |
| Lactose, USP | 106 | 123 |
| Corn Starch, Food Grade | 40 | 70 |
| Magnesium Stearate, NF | 4 | 7 |
| | 200 | 300 |

EXAMPLE XLIII

Lung Inhaler

| Ingredient | | mg/container |
|---|---|---|
| 8,13-epoxy-1α,6β,7β-trihydroxy-labd-14-en-11-one, 7-acetate | | 10.0 |
| Oleic Acid | | 1.0 |
| Fluorotrichloromethane | | 4,739.0 |
| Dichlorodifluoromethane | | 12,250.0 |
| | to make | 17,000.0 |

Add oleic acid to previously cooled fluorotrichloromethane and mix with a high sheer mixer. While mixing, add the required amount of active ingredient and continue mixing until homogeneous. If necessary, adjust the suspension to the required weight with fluorotrichloromethane. Meter the required amount of suspension into each can. Crimp the valves onto the can. Pressure fill through valve required amount of dichlorodifluoromethane to deliver approximately 50 doses per container.

CLAIMS for designated countries other than Austria

1. A pharmaceutical composition for eliciting an antiallergic and/or bronchodilatory effect, which comprises a compound of general formula I

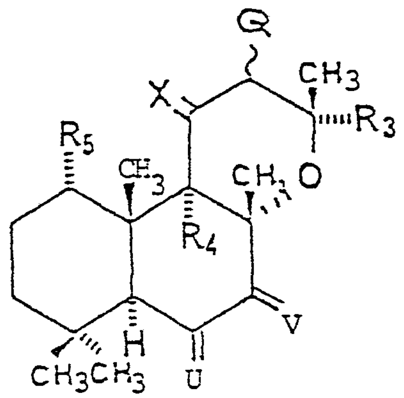

I

including the 5,6 and 6,7 dehydro derivatives, in the free form or in the form of its solvate,

wherein U is =O or $(H,R_1)$;

V is =O or $(H,R_2)$;

wherein $R_1$ is H or $OR_{11}$, wherein $R_{11}$ is H, carboxylic acyl having from 1 to 6 carbon atoms or

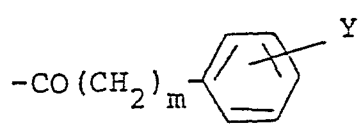

wherein m is 0, 1, 2 or 3 and Y is hydrogen, halogen, alkyl having from 1 to 6 carbon atoms, alkoxy having from 1 to 6 carbon atoms, alkylthio having from 1 to 6 carbon atoms, OH; $CF_3$, $NO_2$, CN, phenyl, benzyl,

phenoxy or $NR_aR_b$ wherein $R_a$ and $R_b$ are the same or different and are H or alkyl having from 1 to 6 carbon atoms;

$R_2$ is H, $OR_{11}$, $OSO_2R'$ or OR', wherein $R_{11}$ is defined as above and R' is alkyl having from 1 to 6 carbon atoms; or when

$R_1$ and $R_2$ are taken together they may form

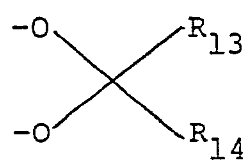

wherein $R_{13}$ and $R_{14}$ may be the same or different and are H, alkyl having from 1 to 6 carbon atoms, alkoxy having from 1 to 6 carbon atoms, alkenyl having from 2 to 6 carbon atoms, alkynyl having from 2 to 6 carbon atoms

or $-(CH_2)_m-$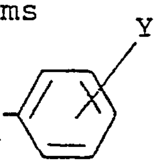 wherein m and Y are as defined above;

$R_3$ is hydrogen, alkyl having from 1 to 10 carbon atoms, $CH_2OH$, CHO, $CO_2R_{15}$ wherein $R_{15}$ is H or alkyl having from 1 to 6 carbon atoms,

$-CH=CR_{16}R_{17}$ wherein $R_{16}$ is H, halogen, alkyl having from 1 to 6 carbon atoms, CN, $\overset{O}{\overset{\|}{C}}(O)_nR_c$ (wherein n is 0 or 1 and $R_c$ is H, alkyl having from 1 to 6 carbon atoms) phenyl or benzyl, $CHOHR_c$ or $C(OR_d)_2R_c$ (wherein $R_c$ is as defined above and $R_d$ is alkyl having

from 1 to 6 carbon atoms), $R_{17}$ is H, alkyl having from 1 to 6 carbon atoms, alkoxy having from 1 to 6 carbon atoms, benzyl, phenyl or halogen;

$-C{\equiv}C-R_{18}$ wherein $R_{18}$ is H, alkyl having from 1 to 12 carbon atoms, alkoxy having from 1 to 6 carbon atoms, $-CH_2$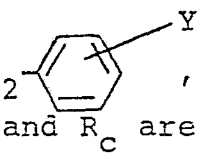, $CO_2R_c$ or 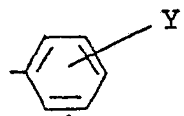 wherein Y and $R_c$ are defined as above;

$-CHOH-C{\equiv}C-R_{19}$ wherein $R_{19}$ is H, alkyl of from 1 to 6 carbon atoms, phenyl or benzyl;

$-CH{=}C{=}CHR_{19}$ or $-CH{=}N-OR_{19}$ wherein $R_{19}$ is defined as above;

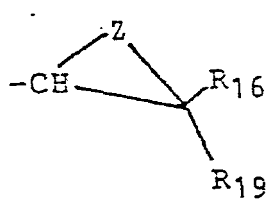

wherein Z is O or S, and $R_{16}$ and $R_{19}$ are defined as above;

$-CH(ZR_{20})_2$ wherein Z is defined as above and $R_{20}$ is alkyl having from 1 to 6 carbon atoms, phenyl, benzyl or the two groups $R_{20}$ may together form $-(CH_2)_p-$ wherein p is 2 or 3;

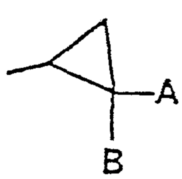

wherein A and B are H, halogen, alkyl having from 1 to 6 carbon atoms, phenyl, benzyl or $\overset{O}{\overset{\|}{C}}(O)_nR_c$ wherein n and $R_c$ are defined as above;

-CH=N-NDE wherein D and E are H, alkyl having from 1 to 6 carbon atoms, benzyl, phenyl, COG, $SO_2G$, or $CO_2G$ wherein G is alkyl having from 1 to 6 carbon atoms, benzyl or phenyl;

$R_4$ is H or OH;

$R_5$ is OH; or when

$R_4$ and $R_5$ are taken together they may form

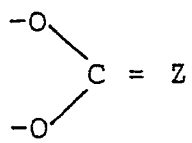 wherein Z is defined as above;

X is O or H/OR" wherein R" is H, carboxylic acyl having from 1 to 6 carbon atoms or trifluoroacetyl;

Q is hydrogen, chlorine or bromine; or Q together with $R_3$ may form a bond between the carbon atoms of positions 12 and 13;

whereby in the 5,6-dehydroderivatives of formula I $R_1$ is hydrogen and in the 6,7-dehydroderivatives of formula I $R_1$ and $R_2$ are hydrogen.

2. A pharmaceutical compositions according to claim 1 characterized in that in formula I

U is $(H,R_1)$ and V is $(H,R_2)$, wherein

$R_1$ and $R_2$ may be the same or different and are hydrogen or $OR_{11}$, wherein $R_{11}$ is defined as in claim 1 and Y is hydrogen;

$R_3$ is $-CH=CHR_{17}$

$-C \equiv CR_{18}$;

$-CHOH-C \equiv C-R_{19}$;

$-CH=C=CHR_{19}$;

$-CH=N-OR_{19}$;

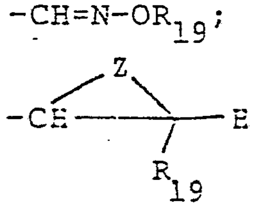 ; 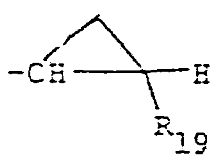 ;

wherein $R_{17}$, $R_{18}$ and $R_{19}$ are hydrogen, alkyl having from 1 to 6 carbon atoms, phenyl or benzyl;

-CH=N-NHD wherein D is hydrogen, alkyl having from 1 to 6 carbon atoms, phenyl, benzyl, $SO_2G$ or $CO_2G$ wherein G is alkyl having from 1 to 6 carbon atoms;

$R_4$ is H or OH;

$R_5$ is OH;

X is O or (H,OH);

Q is hydrogen or chlorine;

the carbon atoms of positions 5,6 and 6,7 are connected by single bonds or

the carbon atoms of positions 5 and 6 are connected by a double bond, in which instance $R_1$ is hydrogen and $R_2$ is preferably $OR_{11}$, wherein $R_{11}$ is defined as in claim 1 and Y is hydrogen.

3. A composition according to claim 1, characterized in that in formula I

U is $(H,R_1)$ and V is $(H,R_2)$ wherein

$R_1$ and $R_2$ may be the same or different and are hydrogen or $OR_{11}$, wherein $R_{11}$ is defined as in claim 1 and Y is hydrogen;

$R_3$ is hydrogen, alkyl having from 1 to 6 carbon atoms, $-CH=CHR_{16}$ or $-C\equiv CR_{18}$ [wherein $R_{16}$ and $R_{18}$ are hydrogen, alkyl having from 1 to 6 carbon atoms, phenyl, benzyl or $CO_2R_c$ wherein $R_c$ is defined as in claim 1];

$R_4$ is H or OH;

$R_5$ is OH;

X is O or (H,OH);

Q is hydrogen or chlorine;

the carbon atoms of positions 5,6 and 6,7 are connected by single bonds or

the carbon atoms of positions 5 and 6 are connected by a double bond, in which instance $R_1$ is H and $R_2$ is preferably $OR_{11}$, wherein $R_{11}$ is defined as in claim 1 and Y is hydrogen;
preferably $R_4$ being H.

4. A composition according to any one of claims 1 to 3, characterized in that in formula I $R_1$ and $R_2$ are $OR_{11}$, wherein $R_{11}$ is H or acyl, preferably containing 1 to 3 carbon atoms and/or $R_3$ is methyl, ethyl or vinyl.

5. A composition according to claim 1, characterized in that the compound of formula I is

forskolin (=7β-acetoxy-8,13-epoxy-1α,6β,9α-trihydroxylabd-14-en-11-one),

7-desacetylforskolin (=8,13-epoxy-1α,6β,7β,9α-tetrahydroxylabd-14-en-11-one);

6-acetoxy-7-desacetylforskolin (=6β-acetoxy-8,13-epoxy-1α,7β,9α-trihydroxylabd-14-en-11-one),

8,13-epoxy-1α,6β,7β-trihydroxy-labd-14-en-11-one 7-acetate;

8,13-epoxy-1α,7β,9α-trihydroxy-labd-5(6)-en-11-one-7-acetate;

8,13-epoxy-1α,6β,7β-trihydroxy-labd-14-en-11-one 6-acetate;

8,13-epoxy-1α,7β,9α-trihydroxy-labda-5(6),14-dien-11-one 7-acetate;

8,13-epoxy-1α,6β,7β-trihydroxy-labd-14-en-11-one;

8,13-epoxy-1α,6β,7β,11β-tetrahydroxy-labd-14-ene;

8,13-epoxy-1α,6β,7β,11β-tetrahydroxy-labd-14-en-7-acetate;

12-chloro-8,13-epoxy-1α,7β-dihydroxy-labda-5(6),14-dien-11-one 7-acetate;

15-nor-8,13-epoxy-1α,6β,7β,9α-tetrahydroxy-labdan-11-one;

15-nor-8,13-epoxy-1α,6β,7β,9α-tetrahydroxy-labdan-11-one-7-acetate;

8,13-epoxy-1α,7β,9α-trihydroxy-labda-5(6),14-dien-11-one-7-propionate, or

8,13-epoxy-1α,7β,9α-trihydroxy-labda-5(6),14-dien-11-one.

6. Pharmaceutical composition for lowering the intraocular pressure containing a compound of formula I as defined in any one of claims 1 to 5, with the exception of 7β-acetoxy-8,13-epoxy-1α,6β,9α-trihydroxylabd-14-en-11-one.

7. Compounds of the general formula I as defined in claim 1, except that:

when $R_4$ is hydrogen, 8,13-epoxy-1α,6β,7β,11α-tetrahydroxylabd-14-ene is excluded and

when $R_4$ is hydroxy or $R_4$ and $R_5$ taken together form the group 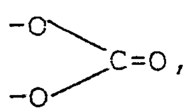, and the carbon atoms of positions (5,6) and (6,7) and (12,13) are connected by single bonds, compounds wherein $R_3$ is $CH=CH_2$, $CH_2CH_3$, CHO or 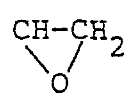

are excluded.

8. A compound according to claim 7, wherein $R_1$ and $R_2$ may be the same or different and are hydrogen or $OR_{11}$, wherein $R_{11}$ is defined as in claim 1 and Y is hydrogen;

$R_3$ is hydrogen, alkyl having from 1 to 6 carbon atoms, $-CH=CHR_{16}$ or $-C{\equiv}CR_{18}$ [wherein $R_{16}$ and $R_{18}$ are hydrogen, alkyl having from 1 to 6 carbon atoms, phenyl, benzyl or $CO_2R_c$ wherein $R_c$ is defined as in claim 1];

$R_5$ is OH;

X is O or (H,OH);

Q is hydrogen or chlorine;

the carbon atoms of positions 5,6 and 6,7 are connected by single bonds or

the carbon atoms of positions 5 and 6 are connected by a double bond, in which instance $R_1$ is H and $R_2$ is preferably $OR_{11}$, wherein $R_{11}$ is defined as in claim 1 and Y is hydrogen.

9. A compound according to claim 7, wherein the carbon atoms of positions 5 and 6 are connected by a double bond, and wherein

$R_1$ is hydrogen and $R_5$ is hydroxy;

or wherein

X is O or α-H/βOH;

Q is H or Cl;

$R_1$ is H;

$R_2$ is $OR_{11}$ ($R_{11}$ being defined as in any one of claims 1 to 5)

$R_3$ is H, $CH_3$, $C_2H_5$ or $CH=CH_2$;

$R_4$ is H or OH; and

$R_5$ is OH;

or wherein

$R_1$ is hydrogen, $R_2$ is $OR_{11}$ wherein $R_{11}$ is acyl having 1 or 2 carbon atoms,

$R_3$ is ethyl or vinyl;

$R_5$ is OH;

X is =O or H/OH; and

Q is hydrogen or Cl.

10. A compound of claim 7, which is

8,13-epoxy-1α,6β,7β-trihydroxy-labd-14-en-11-one 7-acetate;

8,13-epoxy-1α,7β,9α-trihydroxy-labd-5(6)-en-11-one-7-acetate;

8,13-epoxy-1α,6β,7β-trihydroxy-labd-14-en-11-one 6-acetate;

8,13-epoxy-1α,7β,9α-trihydroxy-labda-5(6),14-dien 11-one 7-acetate;

8,13-epoxy-1α,6β,7β-trihydroxy-labd-14-en-11-one;

8,13-epoxy-1α,6β,7β,11β-tetrahydroxy-labd-14-ene;

8,13-epoxy-1α,6β,7β,11β-tetrahydroxy-labd-14-en-7-acetate;

12-chloro-8,13-epoxy-1α,7β-dihydroxy-labda-5(6), 14-dien-11-one 7-acetate;

15-nor-8,13-epoxy-1α,6β,7β,9α-tetrahydroxy-labdan-11-one;

15-nor-8,13-epoxy-1α,6β,7β,9α-tetrahydroxy-labdan-11-one-7-acetate;

8,13-epoxy-1α,7β,9α-trihydroxy-labd-5(6),14-dien-11-one-7-propionate; or

8,13-epoxy-1α,7β,9α-trihydroxy-labda-5(6),14-dien-11-one.

11. Process for the preparation of a compound of formula I as defined in any one of claims 7 to 10, characterized in that forskolin or an appropriate derivative thereof is subjected to at least one of the following processes:

for the preparation of

-) compounds wherein U is =O: oxidizing the corresponding compound wherein U is (H,OH);

-) compounds wherein V is =O: oxidizing the corresponding compound wherein V is (H,OH);

-) compounds wherein U is and/or V is $(H,OR_{11})$, wherein $R_{11}$ is acyl (as defined above): acylation of a corresponding compound wherein $R_{11}$ is hydrogen;

-) compounds wherein U and/or V is (H,OH) and/or $R_4$ and $R_5$ are OH: hydrolysis of the corresponding ester and/or the group

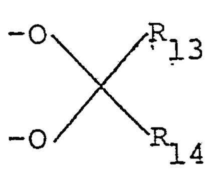 and/or 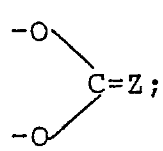

-) compounds wherein U and/or V are (H,H): reduction of the corresponding compound wherein U and/or V are =O;

-) compounds wherein V is $(H,OSO_2R')$, wherein R' is alkyl having from 1 to 6 carbon atoms: reacting a corresponding compound wherein V is (H,OH) with a reactive derivative of $R'SO_3H$;

-) compounds wherein V is (H,OR') wherein R' is alkyl having from 1 to 6 carbon atoms: reacting a corresponding compound, wherein V is (H,OH) with the appropriate alkylhalogenide (R'hal);

-) compounds wherein $R_1$ and $R_2$ together form

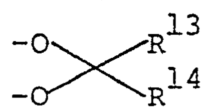

(wherein $R_{13}$ and $R_{14}$ are defined as above): reacting a corresponding compound wherein $R_1=R_2=OH$ with an aldehyde or ketone;

-) compounds wherein the carbon atoms of positions 5,6 or 6,7 are connected by a double bond: dehydration of the corresponding compound wherein said positions are connected by single bonds and U and/or V are (H,OH) or a reactive derivative thereof;

-) compounds wherein the carbon atoms of positions 12 and 13 are connected by a double bond: removing $QR_3$ from a corresponding compound, wherein Q is H and $R_3$ is for example $-CH=NOR_{19}$ wherein $R_{19}$ is preferably hydrogen;

-) compounds wherein $R_4$ is hydrogen: treatment of the corresponding 1,9-carbonate or 1,9-thiocarbonate with zinc in acetic acid;

-) compounds wherein $R_4$ and $R_5$ taken together form 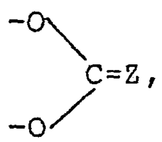, wherein Z is O or S: converting the corresponding compound wherein $R_4$=$R_5$=OH by means of phosgen, carbonyl diimidazole or thiocarbonyl diimidazole, respectively;

-) compounds wherein X is (H,OH): reduction of a compound wherein X is oxo;

-) compounds wherein Q is chlorine or bromine: chlorination or bromination of a corresponding compound wherein Q is hydrogen;

-) compounds wherein $R_3$ is hydroger and X is (H,OH): hydrogenation of a corresponding compound wherein X is O and the carbon atoms of positions 12 and 13 are connected by a double bond;

-) compounds wherein $R_3$ is ethyl: hydrogenation of the corresponding compound wherein $R_3$ is vinyl;

-) compounds wherein $R_3$ is methyl: desulfurizing a corresponding compound wherein

$R_3$ is 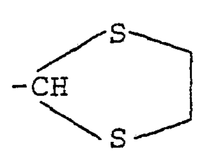 ;

-) compounds wherein $R_3$ is $CH_2OH$: reduction of a corresponding compound wherein $R_3$ is CHO;

-) compounds wherein $R_3$ is CHO: ozonolysis of the corresponding compound wherein $R_3$ is vinyl;

-) compounds wherein $R_3$ is $CO_2R_{15}$: oxidizing a corresponding compound wherein $R_3$ is CHO followed by esterification if $R_{15}$ is alkyl having 1 to 6 carbon atoms;

-) compounds wherein $R_3$ is $-CH=CR_{16}R_{17}$: treating a corresponding compound wherein $R_3$ is CHO with a Wittig reagent, $\emptyset_3P=CR_{16}R_{17}$;

-) compounds wherein $R_3$ is $C\equiv C-R_{18}$: treating a compound wherein $R_3$ is CHO with a reagent $\emptyset_3\overset{+}{P}CHBrR_{18}$ to produce a bromine containing intermediate which may be dehydrobrominated to produce the desired product;

-) compounds wherein $R_3$ is $C\equiv CH$: addition of bromine to a corresponding compound wherein $R_3$ is $-CH=CH_2$ followed by didehydrobromination;

-) compounds wherein $R_3$ is $-CHOH-C\equiv C-R_{19}$: treatment of a compound wherein $R_3$ is CHO with $MC\equiv C-R_{19}$, wherein M is a suitable metal such as lithium;

-) compounds wherein $R_3$ is $-CH=C=CHR_{19}$: esterifying a corresponding compound wherein $R_3$ is $-CHOH-C\equiv C-R_{19}$ and treating the so obtained ester with aluminum chloride;

-) compounds wherein $R_3$ is $-CH=N-OR_{19}$: treatment of a compound wherein $R_3$ is CHO with $H_2N-OR_{19}$;

-) compounds wherein $R_3$ is 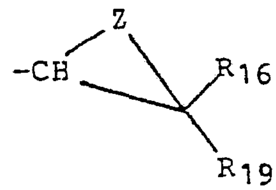 :

treatment of a compound wherein $R_3$ is $-CH=CR_{16}R_{19}$ with a peracid to produce compounds wherein Z is oxygen and, if desired, afterwards treatment with potassium isothiocyanate;

-) compounds wherein $R_3$ is $-CH(ZR_{20})_2$: treatment of a compound wherein $R_3$ is CHO with an alcohol or thiol;

-) compounds wherein $R_3$ is 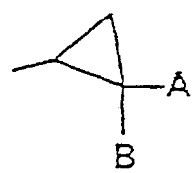 :

addition of a dihalocarbene to a compound wherein $R_3$ is $-CH=CH_2$ or addition of a methylene carbene to a compound wherein $R_3$ is -CH=CAB;

-) compounds wherein $R_3$ is cyclopropyl: reaction of a compound wherein $R_3$ is $-CH=CH_2$ with diazomethane;

-) compounds wherein $R_3$ is -CH=N-NDE: treating a compound wherein $R_3$ is CHO with a compound having the formula $H_2N$-NDE.

CLAIMS for Austria

1. Process for the preparation of a pharmaceutical composition eliciting an antiallergic and/or bronchodilatory effect, characterized in that a compound of general formula I

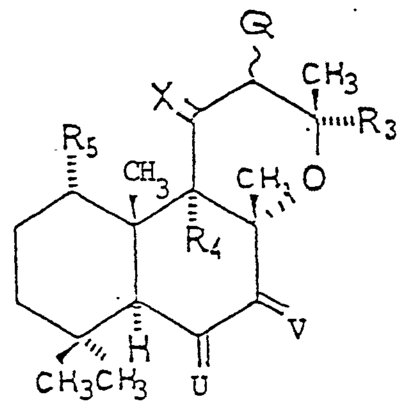

I

including the 5,6 and 6,7 dehydro derivatives, in the free form or in the form of its solvate,

wherein U is =O or $(H,R_1)$;

V is =O or $(H,R_2)$;

wherein $R_1$ is H or $OR_{11}$, wherein $R_{11}$ is H, carboxylic acyl having from 1 to 6 carbon atoms or

$-CO(CH_2)_m-$ 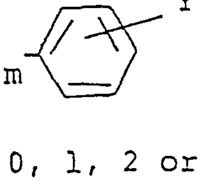

wherein m is 0, 1, 2 or 3 and Y is hydrogen, halogen, alkyl having from 1 to 6 carbon atoms, alkoxy having from 1 to 6 carbon atoms, alkylthio having from 1 to 6 carbon atoms, OH; $CF_3$, $NO_2$, CN, phenyl, benzyl,

phenoxy or $NR_aR_b$ wherein $R_a$ and $R_b$ are the same or different and are H or alkyl having from 1 to 6 carbon atoms;

$R_2$ is H, $OR_{11}$, $OSO_2R'$ or OR', wherein $R_{11}$ is defined as above and R' is alkyl having from 1 to 6 carbon atoms; or when

$R_1$ and $R_2$ are taken together they may form

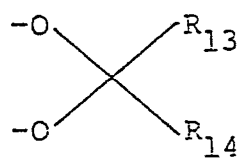

wherein $R_{13}$ and $R_{14}$ may be the same or different and are H, alkyl having from 1 to 6 carbon atoms, alkoxy having from 1 to 6 carbon atoms, alkenyl having from 2 to 6 carbon atoms, alkynyl having from 2 to 6 carbon atoms

or $-(CH_2)_m-$ 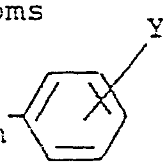 wherein m and Y are as defined above;

$R_3$ is hydrogen, alkyl having from 1 to 10 carbon atoms, $CH_2OH$, CHO, $CO_2R_{15}$ wherein $R_{15}$ is H or alkyl having from 1 to 6 carbon atoms,

$-CH{=}CR_{16}R_{17}$ wherein $R_{16}$ is H, halogen, alkyl having from 1 to 6 carbon atoms, CN, $\overset{\overset{O}{\|}}{C}(O)_nR_c$ (wherein n is 0 or 1 and $R_c$ is H, alkyl having from 1 to 6 carbon atoms) phenyl or benzyl, $CHOHR_c$ or $C(OR_d)_2R_c$ (wherein $R_c$ is as defined above and $R_d$ is alkyl having

from 1 to 6 carbon atoms), $R_{17}$ is H, alkyl having from 1 to 6 carbon atoms, alkoxy having from 1 to 6 carbon atoms, benzyl, phenyl or halogen;

$-C\equiv C-R_{18}$ wherein $R_{18}$ is H, alkyl having from 1 to 12 carbon atoms, alkoxy having from 1 to 6 carbon atoms, $-CH_2-$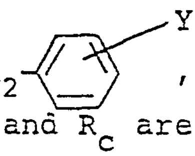, $CO_2R_c$ or 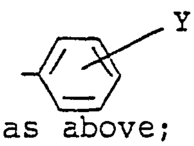 wherein Y and $R_c$ are defined as above;

$-CHOH-C\equiv C-R_{19}$ wherein $R_{19}$ is H, alkyl of from 1 to 6 carbon atoms, phenyl or benzyl;

$-CH=C=CHR_{19}$ or $-CH=N-OR_{19}$ wherein $R_{19}$ is defined as above;

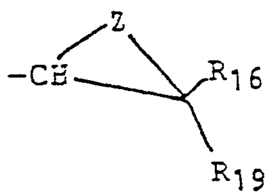

wherein Z is O or S, and $R_{16}$ and $R_{19}$ are defined as above;

$-CH(ZR_{20})_2$ wherein Z is defined as above and $R_{20}$ is alkyl having from 1 to 6 carbon atoms, phenyl, benzyl or the two groups $R_{20}$ may together form $-(CH_2)_p-$ wherein p is 2 or 3;

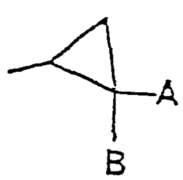

wherein A and B are H, halogen, alkyl having from 1 to 6 carbon atoms, phenyl, benzyl or $\overset{O}{\overset{\|}{C}}(O)_nR_c$ wherein n and $R_c$ are defined as above;

-CH=N-NDE wherein D and E are H, alkyl having from 1 to 6 carbon atoms, benzyl, phenyl, COG, $SO_2G$, or $CO_2G$ wherein G is alkyl having from 1 to 6 carbon atoms, benzyl or phenyl;

$R_4$ is H or OH;

$R_5$ is OH; or when

$R_4$ and $R_5$ are taken together they may form

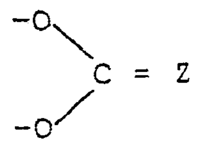

wherein Z is defined as above;

X is O or H/OR" wherein R" is H, carboxylic acyl having from 1 to 6 carbon atoms or trifluoroacetyl;

Q is hydrogen, chlorine or bromine; or Q together with $R_3$ may form a bond between the carbon atoms of positions 12 and 13;

whereby in the 5,6-dehydroderivatives of formula I $R_1$ is hydrogen and in the 6,7-dehydroderivatives of formula I $R_1$ and $R_2$ are hydrogen,

is brought into a form suitable for pharmaceutical administration.

2. Process according to claim 1, characterized in that in formula I

U is $(H,R_1)$ and V is $(H,R_2)$, wherein

$R_1$ and $R_2$ may be the same or different and are hydrogen or $OR_{11}$, wherein $R_{11}$ is defined as in claim 1 and Y is hydrogen;

$R_3$ is $-CH=CHR_{17}$

$-C\equiv CR_{18}$;

$-CHOH-C\equiv C-R_{19}$;

$-CH=C=CHR_{19}$;

$-CH=N-OR_{19}$;

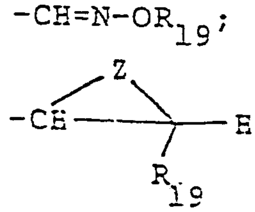

;

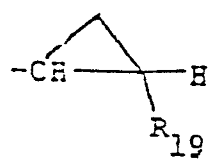

;

wherein $R_{17}$, $R_{18}$ and $R_{19}$ are hydrogen, alkyl having from 1 to 6 carbon atoms, phenyl or benzyl;

$-CH=N-NHD$ wherein D is hydrogen, alkyl having from 1 to 6 carbon atoms, phenyl, benzyl, $SO_2G$ or $CO_2G$ wherein G is alkyl having from 1 to 6 carbon atoms;

$R_4$ is H or OH;

$R_5$ is OH;

X is O or (H,OH);

Q is hydrogen or chlorine;

the carbon atoms of positions 5,6 and 6,7 are connected by single bonds or

the carbon atoms of positions 5 and 6 are connected by a double bond, in which instance $R_1$ is hydrogen and $R_2$ is preferably $OR_{11}$, wherein $R_{11}$ is defined as in claim 1 and Y is hydrogen.

3. Process according to claim 1, characterized in that in formula I

U is $(H,R_1)$ and V is $(H,R_2)$ wherein

$R_1$ and $R_2$ may be the same or different and are hydrogen or $OR_{11}$, wherein $R_{11}$ is defined as in claim 1 and Y is hydrogen;

$R_3$ is hydrogen, alkyl having from 1 to 6 carbon atoms, $-CH=CHR_{16}$ or $-C{\equiv}CR_{18}$ [wherein $R_{16}$ and $R_{18}$ are hydrogen, alkyl having from 1 to 6 carbon atoms, phenyl, benzyl or $CO_2R_c$ wherein $R_c$ is defined as in claim 1];

$R_4$ is H or OH;

$R_5$ is OH;

X is O or (H,OH);

Q is hydrogen or chlorine;

the carbon atoms of positions 5,6 and 6,7 are connected by single bonds or

the carbon atoms of positions 5 and 6 are connected by a double bond, in which instance $R_1$ is H and $R_2$ is preferably $OR_{11}$, wherein $R_{11}$ is defined as in claim 1 and Y is hydrogen; preferably $R_4$ being H.

4. Process according to any one of claims 1 to 3, characterized in that in formula I $R_1$ and $R_2$ are $OR_{11}$, wherein $R_{11}$ is H or acyl, preferably containing 1 to 3 carbon atoms and/or $R_3$ is methyl, ethyl or vinyl.

5. Process according to claim 1, characterized in that the compound of formula I is

forskolin (=7β-acetoxy-8,13-epoxy-1α,6β,9α-trihydroxylabd-14-en-11-one),

7-desacetylforskolin (=8,13-epoxy-1α,6β,7β,9α-tetrahydroxylabd-14-en-11-one);

6-acetoxy-7-desacetylforskolin (=6β-acetoxy-8,13-epoxy-1α,7β,9α-trihydroxylabd-14-en-11-one),

8,13-epoxy-1α,6β,7β-trihydroxy-labd-14-en-11-one 7-acetate;

8,13-epoxy-1α,7β,9α-trihydroxy-labd-5(6)-en-11-one-7-acetate;

8,13-epoxy-1α,6β,7β-trihydroxy-labd-14-en-11-one 6-acetate;

8,13-epoxy-1α,7β,9α-trihydroxy-labda-5(6),14-dien-11-one 7-acetate;

8,13-epoxy-1α,6β,7β-trihydroxy-labd-14-en-11-one;

8,13-epoxy-1α,6β,7β,11β-tetrahydroxy-labd-14-ene;

8,13-epoxy-1α,6β,7β,11β-tetrahydroxy-labd-14-en 7-acetate;

12-chloro-8,13-epoxy-1α,7β-dihydroxy-labda-5(6),14-dien-11-one 7-acetate;

15-nor-8,13-epoxy-1α,6β,7β,9α-tetrahydroxy-labdan-11-one;

15-nor-8,13-epoxy-1α,6β,7β,9α-tetrahydroxy-labdan-11-one-7-acetate;

8,13-epoxy-1α,7β,9α-trihydroxy-labda-5(6),14-dien-11-one-7-propionate, or

8,13-epoxy-1α,7β,9α-trihydroxy-labda-5(6),14-dien-11-one.

6. Process for the preparation of a pharmaceutical composition for lowering the intraocular pressure characterized in that a compound of formula I as defined in any one of claims 1 to 5, with the exception of 7β-acetoxy-8,13-epoxy-1α,6β,9α-trihydroxylabd-14-en-11-one is brought into a form suitable for ophthalmic administration.

7. Process for the preparation of a compound of the general formula I as defined in claim 1, except that:

when $R_4$ is hydrogen, 8,13-epoxy-1α,6β,7β,11α-tetrahydroxylabd-14-ene is excluded and

when $R_4$ is hydroxy or $R_4$ and $R_5$ taken together form the group 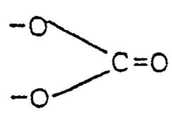, and the carbon atoms of positions (5,6) and (6,7) and (12,13) are connected by single bonds, compounds wherein $R_3$ is $CH=CH_2$, $CH_2CH_3$, CHO or 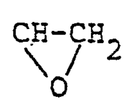

are excluded,

characterized in that forskolin or an appropriate derivative thereof is subjected to at least one of the following processes:

for the preparation of

-) compounds wherein U is =O: oxidizing the corresponding compound wherein U is (H,OH);

-) compounds wherein V is =O: oxidizing the corresponding compound wherein V is (H,OH);

-) compounds wherein U is and/or V is $(H,OR_{11})$, wherein $R_{11}$ is acyl (as defined above): acylation of a corresponding compound wherein $R_{11}$ is hydrogen;

-) compounds wherein U and/or V is (H,OH) and/or $R_4$ and $R_5$ are OH: hydrolysis of the corresponding ester and/or the group

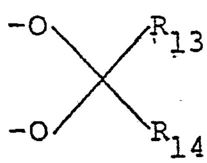 and/or 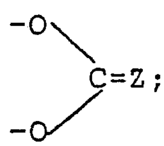

-) compounds wherein U and/or V are (H,H): reduction of the corresponding compound wherein U and/or V are =O;

-) compounds wherein V is $(H,OSO_2R')$, wherein R' is alkyl having from 1 to 6 carbon atoms: reacting a corresponding compound wherein V is (H,OH) with a reactive derivative of $R'SO_3H$;

-) compounds wherein V is (H,OR') wherein R' is alkyl having from 1 to 6 carbon atoms: reacting a corresponding compound, wherein V is (H,OH) with the appropriate alkylhalogenide (R'hal);

-) compounds wherein $R_1$ and $R_2$ together form

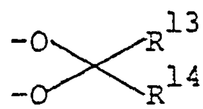

(wherein $R_{13}$ and $R_{14}$ are defined as above): reacting a corresponding compound wherein $R_1=R_2=OH$ with an aldehyde or ketone;

-) compounds wherein the carbon atoms of positions 5,6 or 6,7 are connected by a double bond: dehydration of the corresponding compound wherein said positions are connected by single bonds and U and/or V are (H,OH) or a reactive derivative thereof;

-) compounds wherein the carbon atoms of positions 12 and 13 are connected by a double bond: removing $QR_3$ from a corresponding compound, wherein Q is H and $R_3$ is for example $-CH=NOR_{19}$ wherein $R_{19}$ is preferably hydrogen;

-) compounds wherein $R_4$ is hydrogen: treatment of the corresponding 1,9-carbonate or 1,9-thiocarbonate with zinc in acetic acid;

-) compounds wherein $R_4$ and $R_5$ taken together form 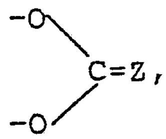 , wherein Z is O or S: converting the corresponding compound wherein $R_4$=$R_5$=OH by means of phosgen, carbonyl diimidazole or thiocarbonyl diimidazole, respectively;

-) compounds wherein X is (H,OH): reduction of a compound wherein X is oxo;

-) compounds wherein Q is chlorine or bromine: chlorination or bromination of a corresponding compound wherein Q is hydrogen;

-) compounds wherein $R_3$ is hydrogen and X is (H,OH): hydrogenation of a corresponding compound wherein X is O and the carbon atoms of positions 12 and 13 are connected by a double bond;

-) compounds wherein $R_3$ is ethyl: hydrogenation of the corresponding compound wherein $R_3$ is vinyl;

-) compounds wherein $R_3$ is methyl: desulfurizing a corresponding compound wherein

$R_3$ is 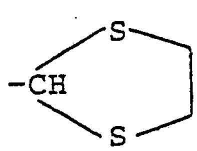 ;

-) compounds wherein $R_3$ is $CH_2OH$: reduction of a corresponding compound wherein $R_3$ is CHO;

-) compounds wherein $R_3$ is CHO: ozonolysis of the corresponding compound wherein $R_3$ is vinyl;

-) compounds wherein $R_3$ is $CO_2R_{15}$: oxidizing a corresponding compound wherein $R_3$ is CHO followed by esterification if $R_{15}$ is alkyl having 1 to 6 carbon atoms;

-) compounds wherein $R_3$ is $-CH{=}CR_{16}R_{17}$: treating a corresponding compound wherein $R_3$ is CHO with a Wittig reagent, $\emptyset_3P{=}CR_{16}R_{17}$;

-) compounds wherein $R_3$ is $C{\equiv}C{-}R_{18}$: treating a compound wherein $R_3$ is CHO with a reagent $\emptyset_3\overset{+}{P}CHBrR_{18}$ to produce a bromine containing intermediate which may be dehydrobrominated to produce the desired product;

-) compounds wherein $R_3$ is $C{\equiv}CH$: addition of bromine to a corresponding compound wherein $R_3$ is $-CH{=}CH_2$ followed by didehydrobromination;

-) compounds wherein $R_3$ is $-CHOH{-}C{\equiv}C{-}R_{19}$: treatment of a compound wherein $R_3$ is CHO with $MC{\equiv}C{-}R_{19}$, wherein M is a suitable metal such as lithium;

-) compounds wherein $R_3$ is $-CH{=}C{=}CHR_{19}$: esterifying a corresponding compound wherein $R_3$ is $-CHOH{-}C{\equiv}C{-}R_{19}$ and treating the so obtained ester with aluminum chloride;

-) compounds wherein $R_3$ is $-CH=N-OR_{19}$: treatment of a compound wherein $R_3$ is CHO with $H_2N-OR_{19}$;

-) compounds wherein $R_3$ is 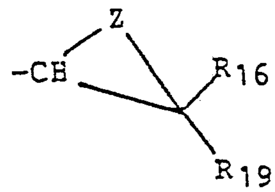 :

treatment of a compound wherein $R_3$ is $-CH=CR_{16}R_{19}$ with a peracid to produce compounds wherein Z is oxygen and, if desired, afterwards treatment with potassium isothiocyanate;

-) compounds wherein $R_3$ is $-CH(ZR_{20})_2$: treatment of a compound wherein $R_3$ is CHO with an alcohol or thiol;

-) compounds wherein $R_3$ is 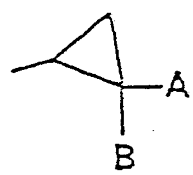 :

addition of a dihalocarbene to a compound wherein $R_3$ is $-CH=CH_2$ or addition of a methylene carbene to a compound wherein $R_3$ is $-CH=CAB$;

-) compounds wherein $R_3$ is cyclopropyl: reaction of a compound wherein $R_3$ is $-CH=CH_2$ with diazomethane;

-) compounds wherein $R_3$ is $-CH=N-NDE$: treating a compound wherein $R_3$ is CHO with a compound having the formula $H_2N-NDE$.

8. A process according to claim 7, characterized in that a compound of formula I is prepared wherein

$R_1$ and $R_2$ may be the same or different and are hydrogen or $OR_{11}$, wherein $R_{11}$ is defined as in claim 1 and Y is hydrogen;

$R_3$ is hydrogen, alkyl having from 1 to 6 carbon atoms, $-CH=CHR_{16}$ or $-C\equiv CR_{18}$ [wherein $R_{16}$ and $R_{18}$ are hydrogen, alkyl having from 1 to 6 carbon atoms, phenyl, benzyl or $CO_2R_c$ wherein $R_c$ is defined as in claim 1];

$R_5$ is OH;

X is O or (H,OH);

Q is hydrogen or chlorine;

the carbon atoms of positions 5,6 and 6,7 are connected by single bonds or

the carbon atoms of positions 5 and 6 are connected by a double bond, in which instance $R_1$ is ~~preferably~~ H and $R_2$ is preferably $OR_{11}$, wherein $R_{11}$ is defined as in claim 1 and Y is hydrogen.

9. A process according to claim 7, characterized in that a compound of formula I is prepared wherein the carbon atoms of positions 5 and 6 are connected by a double bond, and wherein

$R_1$ is hydrogen and $R_5$ is hydroxy;

or wherein

X is O or α-H/βOH;

Q is H or Cl;

$R_1$ is H;

$R_2$ is $OR_{11}$ ($R_{11}$ being defined as in any one of claims 1 to 5)

$R_3$ is H, $CH_3$, $C_2H_5$ or $CH=CH_2$;

$R_4$ is H or OH; and

$R_5$ is OH;

or wherein

$R_1$ is hydrogen, $R_2$ is $OR_{11}$ wherein $R_{11}$ is acyl having 1 or 2 carbon atoms,

$R_3$ is ethyl or vinyl;

$R_5$ is OH;

X is =O or H/OH; and

Q is hydrogen or Cl.

10. A process according to claim 7, characterized in that

8,13-epoxy-1α,6β,7β-trihydroxy-labd-14-en-11-one 7-acetate;

8,13-epoxy-1α,7β,9α-trihydroxy-labd-5(6)-en-11-one-7-acetate;

8,13-epoxy-1α,6β,7β-trihydroxy-labd-14-en-11-one 6-acetate;

8,13-epoxy-1α,7β,9α-trihydroxy-labda-5(6),14-dien 11-one 7-acetate;

8,13-epoxy-1α,6β,7β-trihydroxy-labd-14-en-11-one;

8,13-epoxy-1α,6β,7β,11β-tetrahydroxy-labd-14-ene;

8,13-epoxy-1α,6β,7β,11β-tetrahydroxy-labd-14-en-7-acetate;

12-chloro-8,13-epoxy-1α,7β-dihydroxy-labda-5(6), 14-dien-11-one 7-acetate;

15-nor-8,13-epoxy-1α,6β,7β,9α-tetrahydroxy-labdan-11-one;

15-nor-8,13-epoxy-1α,6β,7β,9α-tetrahydroxy-labdan-11-one-7-acetate;

8,13-epoxy-1α,7β,9α-trihydroxy-labd-5(6),14-dien-11-one-7-propionate; or

8,13-epoxy-1α,7β,9α-trihydroxy-labda-5(6),14-dien-11-one

is prepared.

European Patent Office

# EUROPEAN SEARCH REPORT

0116713

Application number

EP 83 11 2756

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,A | US-A-4 134 986 (B.S. BAJWA et al.)<br>* Columns 1-11 *<br>--- | 1,6,7, 11 | C 07 D 311/92<br>C 07 D 409/04 |
| D,A | GB-A-1 589 326 (HOECHST)<br>* Pages 5,6 and claims *<br>--- | 1,6,7 | |
| D,X | ARZNEIMITTEL-FORSCHUNG / DRUG RES., vol. 31 (II), no. 8, 1981, pages 1248-1250, Aulendorf, DE<br>H. METZGER et al.: "The positive inotropic-acting forskolin, a potent adenylatecyclase activator" * Pages 1248-1250 *<br>--- | 1,5 | |
| D,A | JOURNAL OF THE CHEMICAL SOCIETY, PERKIN TRANS. I, vol. 3, 1982, page 767, London, GB<br>S.V. BHAT et al.: "Reactions of forskolin, a biologically active diterpenoid from Coleus forskhohlii" * Pages 676,768-771, experimental" *<br>--- | 7,11 | TECHNICAL FIELDS SEARCHED (Int. Cl. 3)<br>C 07 D 311/00 |
| X | TETRAHEDRON LETTERS, no. 19, 1977, pages 1669-1672, Pergamon Press, Oxford, GB<br>S.V. BHAT et al.: "Structures and stereochemistry of new labdane diterpenoids from coleus forskohlii briq" * Pages 669-670 *<br>--- -/- | 7,11 | |

The present search report has been drawn up for all claims

| Place of search | Date of completion of the search | Examiner |
|---|---|---|
| THE HAGUE | 06-04-1984 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

0116713
Application number

EP 83 11 2756

Page 2

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int Cl. 3) |
| X | CHEMICAL ABSTRACTS, vol. 89, no. 24, 1978, page 671, no. 24556z, Columbus, Ohio, US J.S. TANDON et al.: "Structure of coleonol, a biologically active diterpene from coleus forskohlii" & INDIAN J. CHEM., SECT. B 1977, 15B(10), 880-3 * Abstract * --- | 1,4,5 | |
| X | CHEMICAL ABSTRACTS, vol. 89, 1978, page 594, no. 163779n, Columbus, Ohio, US J.S. TANDON et al.: "Structures of three new diterpenes, coleonol B, coleonol C and deoxycoleonol isolated from coleus forskohlii" & INDIAN J. CHEM., SECT. B 1978, 16B(5), 341-5 * Compound I * ----- | 7 | |
| | | | TECHNICAL FIELDS SEARCHED (Int. Cl. 3) |

The present search report has been drawn up for all claims

| Place of search | Date of completion of the search | Examiner |
|---|---|---|
| THE HAGUE | 06-04-1984 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82